(19)

Europäisches
Patentamt
European
Patent Office
Office européen
des brevets

(11) **EP 1 956 371 A2**

(12) **EUROPEAN PATENT APPLICATION**

(43) Date of publication:
**13.08.2008 Bulletin 2008/33**

(51) Int Cl.:
**G01N 33/48** (2006.01)

(21) Application number: **07255006.4**

(22) Date of filing: **21.12.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **21.12.2006 US 876402 P**
**20.11.2007 US 943226**

(71) Applicants:
• **LifeScan, Inc.**
**Milpitas, CA 95035 (US)**
• **UNIVERSITY OF VIRGINIA PATENT FOUNDATION**
**Charlottesville, VA 22902 (US)**

(72) Inventors:
• **Kovatchev, Boris P.**
**Charlottesville, VA 22901 (US)**
• **Price, David**
**Pleasonton, CA 94566 (US)**
• **Otto, Erik**
**San Francisco, CA 94115 (US)**
• **Coulson, Alan**
**Nairn, Scotland IV12 4SA (GB)**

(74) Representative: **Tunstall, Christopher Stephen et al**
**Carpmaels & Ransford**
**43-45 Bloomsbury Square**
**London WC1A 2RA (GB)**

(54) **Systems, methods and computer program codes for recognition of patterns of hyperglycemia and hypoglycemia, increased glucose variability, and ineffective self-monitoring in diabetes**

(57) A method, system, and computer program product related to the maintenance of optimal control of diabetes, and is directed to predicting patterns of hypoglycemia, hyperglycemia, increased glucose variability, and insufficient or excessive testing for the upcoming period of time, based on blood glucose readings collected by a self-monitoring blood glucose device. The method, system, and computer program product pertain directly to the enhancement of existing home blood glucose monitoring devices, by introducing an intelligent data interpretation component capable of predicting and alerting the user to periods of increased risk for hyperglycemia, hypoglycemia, increased glucose variability, and ineffective testing, and to the enhancement of emerging self-monitoring blood glucose devices by the same features. With these predictions the diabetic can take steps to prevent the adverse consequences associated with hyperglycemia, hypoglycemia, and increased glucose variability.

**FIG. 2**

**EP 1 956 371 A2**

**Description**

**RELATED APPLICATIONS**

The present patent application claims priority from U.S. Provisional Application Serial No. 60/876,402, filed December 21, 2006, entitled "Systems, Methods and Computer Program Codes for Recognition of Patterns of Hyperglycemia and Hypoglycemia, Increased Glucose Variability, and Ineffective Self-Monitoring in Diabetes," the entire disclosure of which is hereby incorporated by reference herein in its entirety.

The present patent application is related to the International Patent Application Serial No. PCT/US2007/000370, filed January 5, 2007, entitled "Method, System, and Computer Program Product for Evaluation of Blood Glucose Variability in Diabetes from Self-Monitoring Data," the entire disclosure of which is hereby incorporated by reference herein in it's entirety.

**TECHNICAL FIELD OF INVENTION**

The present system relates generally to the art of glucose monitoring, and more particularly to hypo- and hyperglycemic risk assessment.

**BACKGROUND OF THE INVENTION**

Bio-behavioral feedback and its critical importance for the control of diabetes is a complex of disorders, characterized by a common final element of hyperglycemia, that arise from, and are determined in their progress by, mechanisms acting at all levels of bio-system organization - from molecular, through hormonal, to human behavior. Intensive treatment with insulin and with oral medication to maintain nearly normal levels of glycemia markedly reduces chronic complications in both Type 1 (34,40) and Type 2 diabetes (T1DM, T2DM, 42), but may risk potentially life-threatening severe hypoglycemia (SH). State-of-the-art but imperfect insulin replacement may reduce warning symptoms and hormonal defenses against hypoglycemia, resulting in cognitive dysfunction, stupor, coma, or sudden death (16,17,31,39). In addition, recent studies suggest that hypoglycemia may trigger *increased insulin sensitivity* (18,32). Therefore, hypoglycemia has been identified as the primary barrier to optimal diabetes management (9,11). People with T1DM and T2DM face a life-long *behaviorally controlled optimization problem* to reduce hyperglycemic excursions and maintain strict glycemic control, without increasing their risk for hypoglycemia.

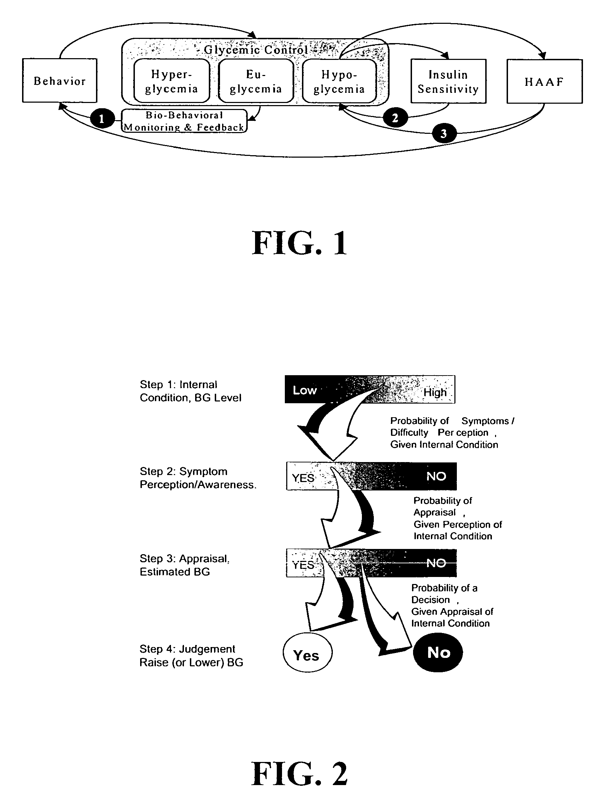

A key to this, as well as many, optimization problems is providing and appropriately utilizing available feedback for the system's status and dynamics. Approached from a systems biology point of view, the task of optimization of T1DM requires study of feedback loops at several bio-system levels: (1) external feedback to the patient altering human behavior; (ii) insulin-glucose interaction, and (iii) hormonal feedback occurring with recurrent hypoglycemia from one hypoglycemic episode to the next, reflected by hypoglycemia-associated autonomic failure (HAAF, 10). **Figure 1** presents these three major feedback loops.

Feedback loop 1 (**Figure 1**), the temporal patterns of glycemia and behavior, is the background of this invention. This process is influenced by many external factors, including the timing and amount of insulin injected, food eaten, physical activity, etc. In other words, blood glucose (BG) fluctuations in diabetes are the measurable result of the action of a complex dynamical system, influenced by many internal and external factors. The macro (human)-level optimization of this system depends on self-treatment behavior. Thus, such an optimization has to be based on feedback utilizing data available in the field, such as self-monitoring of blood glucose (SMBG), knowledge of $HbA_{1c}$, and monitoring of symptoms and self-treatment practices. These macro-level information sources are at various stages of development and clinical acceptance, with $HbA_{1c}$ assays and SMBG now routine practice:

$HbA_{1c}$: This classic marker of average glycemic status (1) has been linked to long-term complications of diabetes, and confirmed as the gold standard for both T1DM and T2DM (36). However, $HbA_{1c}$ has repeatedly been proven to be an ineffective assessment of patients' extreme glucose fluctuations. The DCCT concluded that only about 8% of SH episodes could be predicted from known variables, including $HbA_{1c}$ (39); later this prediction was improved to 18% by a structural equation model using history of SH, awareness, and autonomic symptom score (15). In our studies $HbA_{1c}$ has never been significantly associated with SH (6,23,27).

SMBG: Contemporary home BG meters offer convenient means for frequent and accurate BG determinations through SMBG (3,7,41). Most meters are capable of storing hundreds of SMBG readings, together with the date and time of each reading, and have interfaces to download these readings into a PC. The meters are usually accompanied by software that has capabilities for basic data analyses (e.g. calculation of mean BG, estimates of the average BG over the previous two weeks, percentages in target, hypoglycemic and hyperglycemic zones, etc.), log of the data, and graphical representation (e.g. histograms, pie charts). However, while these devices provide information about current status of BG at a point in time, none of them provide assessment of patients' overall glycemic control, BG patterns and trends, or self-treatment effectiveness when a BG result is given. In a series of studies we have shown that specific analysis of SMBG data, the low BG Index (LBGI), could capture long-term trends towards increased risk for hypoglycemia (23,24,29) and could identify 24-hour periods of increased risk for hypoglycemia (20). These analyses were based on recognition of a specific asymmetry of the BG measurement scale and on a nonlinear transformation correcting this asymmetry (22,25). Since our first announcement (6), we refined and further validated our methods and presented a structured theory of Risk Analysis of BG Data (28). This theory became a basis for our algorithms using SMBG to comprehensively evaluate glycemic control in T1DM (21, 27).

Monitoring and Assessment of Behavior: In order to formally describe the behavioral self-treatment process we created the Stochastic Model of Self-Regulation Behavior, which gives a mathematical description to the feedback pattern internal condition - symptom perception/awareness - appraisal - self-regulation decision (26, **Figure 2**). The idea behind this model is that internal events, such as low (or high) BG episodes, are followed by self-regulation behavioral sequences that, if inappropriate, could lead the patient to SH, or extreme hyperglycemia, and if appropriate, lead to avoidance of these extreme situations. Four sequential steps, internal condition (e.g. low/high BG) - symptom perception/ awareness - appraisal/judgment/self-regulation decision, can be distinguished. The steps are linked by a continuum of possible pathways, i.e. there are a variety of possible perceptions of a low/high BG level as shown in Step 1 to Step 2 of **Figure 2**, there is no single possible appraisal of a perception as shown in Step 2 to Step 3 of **Figure 2**, and there is no uniquely predetermined decision that follows an appraisal of the situation as shown in Step 3 to Step 4 of **Figure 2**. The model is stochastic, that is transitions from one step to the next are represented by families of transition probabilities (26, 33). The model of self-treatment behavior provides the theoretical base for this inventions disclosure - appropriate feedback to the person will change the perception and awareness of the person, thereby leading to better self-regulation decisions and improved diabetes control. This premise is experimentally confirmed by the studies described in the next paragraph.

Utility of Behavioral Interventions: Several studies have documented that the avoidance of low BG events (<70 mg/dl) for a few weeks can improve symptom perception and reverse hypoglycemia unawareness (8,12,13,14). While such an intervention has promise for reducing SH risk, it needs close patient monitoring to ensure that metabolic control is not jeopardized (2). We have previously developed BGAT, a well-documented, effective, psycho-behavioral intervention for people with T1DM. Its many positive effects include improvement in BG detection, BG profiles, psychosocial status, knowledge, decision-making, and reduced life-threatening events such as severe hypo- and hyperglycemia (4,5). Additionally, we have shown that BGAT improves low BG detection in *both* patients with intact hypoglycemic symptoms and those with reduced awareness. Researchers at the Joslin Clinic found that BGAT preserved counterregulation integrity in patients undergoing intensive insulin therapy (19).

An aspect of various embodiments of the present invention focuses on, but not limited thereto, Feedback loop # 1 (**Figure 1**) - temporal patterns of glycemia and behavior. A premise is that to enable behavioral changes via algorithmic recognition of idiosyncratic temporal patterns of glycemia and self-testing. In particular, patterns of hyperglycemia and hypoglycemia, increased glucose variability, and ineffective self-monitoring are recognized, and messages are conveyed back to the person in real time.

As postulated by the stochastic model of self-treatment behavior **(Figure 2),** this in turn prompts a sequence of increased awareness, improved appraisal, and better self-treatment behaviors, which result in improved glycemic control. Of particular significance relative to this invention is the timing of the idiosyncratic feedback. An aspect of various embodiments of this invention, feedback is given relative to the identification of temporal patterns of glycemia and self-testing to the patient immediately before the timeframe of the day pertinent to the messaging. Preferably the messaging is given when the patient is testing their BG, and the messaging should be about patterns typically occurring the subsequent time period, which could be any where from about 2 to 8 hours long. This allows the patient to take corrective actions immediately before the time period for which information is given in order to prevent the reoccurrence of any deleterious glycemic patterns. The timing of insight and the resulting motivation for action is anticipated to have a strong impact on improving self-management awareness and behavior and ultimately improve patient glycemic control.

## BRIEF SUMMARY OF INVENTION

An aspect of various embodiments of the present invention consists of, but not limited thereto, four methods and algorithms for identifying patterns of: (i) hyperglycemia; (ii) hypoglycemia, (iii) increased glucose variability, and (iv) ineffective self-testing. The methods use routine SMBG data collected over a period of 2-6 weeks. SMBG is defined as episodic non-automated determination (typically 2 or more times per day) of blood glucose obtained in a diabetic patients' natural environment. A user, subject or patient may monitor oneself or rely on the assistance of others, e.g. a layperson, acquaintance, clinician, other medical professional, etc.

Various embodiments of the present invention may pertain directly to, among other things, the following:

■ Enhancement of existing SMBG devices by introducing an intelligent data interpretation component capable of evaluating temporal glucose patterns and enabling of future SMBG devices by the same features;

■ Enhancement by the same features of hand-held devices (personal digital assistants, PDA, mobile phones / e-mail devices, insulin pumps etc...) intended to assist diabetes management;

■ Enhancement by the same features of software that retrieve SMBG data - such software is produced by virtually every manufacturer of home BG monitoring devices and is customarily used by patients and health care providers for interpretation of SMBG data. The software can reside on patients personal computers, or be used via Internet portal;

■ Evaluation of the effectiveness of various treatments for diabetes (insulin, variability lowering medications, such as pramlintide and exenatide).

■ Evaluation of the effectiveness new insulin delivery devices (insulin pumps), or the future closed-loop diabetes control systems.

One aspect of the invention includes a system, method, and computer program product for identifying patterns of hyperglycemia, defined as glucose averages within certain periods of time that exceed certain high glucose thresholds.

Another aspect of the invention includes a system, method, and computer program product for identifying patterns of hypoglycemia, defined as glucose averages within certain periods of time that exceed certain low glucose thresholds.

Another aspect of the invention includes a system, method, system, and computer program for identifying patterns of increased glucose variability, defined as certain periods of time in which the Average Daily Risk Range (ADRR), Standard Deviation, or other measure of blood glucose variability exceed certain thresholds. The ADRR is described in detail in a previously filed International Patent Application Serial No. PCT/US2007/000370, filed January 5, 2007, entitled "Method, System and Computer Program Product for Evaluation of Blood Glucose Variability in Diabetes from Self-Monitoring Data" and see recent publication (30).

A fourth aspect of the invention includes a system, method, system, and computer program for identifying patterns of ineffective self-testing. Similar patterns have been described in a previous patent application (See PCT International Application No. PCT/US2003/025053, filed on August 8, 2003) as a system of sample selection criteria for the evaluation of HbA1c from SMBG data.

The four pattern recognition methods use both population thresholds <u>and</u> individual thresholds adjusted for the glycemic status of the person. These four aspects of the invention can be integrated together to provide information about the timing during the day of risk for hyperglycemia, risk of hypoglycemia, risk of increased glucose variability, and ineffective testing of an individual with diabetes. Such information can be presented in addition to the information obtained and displayed by previously disclosed methods evaluating HbA1c, long-term and imminent risk for hypoglycemia, and overall glucose variability or other methods of evaluating patient status.

An aspect of an embodiment of the present invention provides a method for identifying and/or predicting patterns of hyperglycemia of a user. The method may compromise: acquiring plurality of SMBG data points; classifying said SMBG data points within periods of time with predetermined durations; evaluating glucose values in each period of time; and indicating risk of hyperglycemia for a subsequent period of time based on said evaluation.

An aspect of an embodiment of the present invention provides a system for identifying and/or predicting patterns of hyperglycemia of a user, wherein the system comprises an acquisition module acquiring a plurality of blood glucose data points and a processor. The processor may be programmed to: classify said SMBG data points within periods of time with predetermined durations; evaluate glucose values in each period of time; and indicate risk of hyperglycemia for a subsequent period of time based on said evaluation.

An aspect of an embodiment of the present invention provides a computer program product comprising a computer useable medium having computer program logic for enabling at least one processor in a computer system to identify and/or predict patterns of hyperglycemia of a user. The computer program logic may comprise: acquiring plurality of SMBG data points; classifying said SMBG data points within periods of time with predetermined durations; evaluating glucose values in each period of time; and indicating risk of hyperglycemia for a subsequent period of time based on said evaluation.

An aspect of an embodiment of the present invention provides a method for identifying and/or predicting patterns of hypoglycemia of a user. The method may compromise: acquiring plurality of SMBG data points; classifying said SMBG data points within periods of time with predetermined durations; evaluating glucose values in each period of time; and indicating risk of hypoglycemia for a subsequent period of time based on said evaluation.

An aspect of an embodiment of the present invention provides a system for identifying and/or predicting patterns of hypoglycemia of a user, wherein the system comprises an acquisition module acquiring a plurality of blood glucose data points and a processor. The processor may be programmed to: classify said SMBG data points within periods of time with predetermined durations; evaluate glucose values in each period of time; and indicate risk of hypoglycemia for a subsequent period of time based on said evaluation.

An aspect of an embodiment of the present invention provides a computer program product comprising a computer useable medium having computer program logic for enabling at least one processor in a computer system to identify and/or predict patterns of hypoglycemia of a user. The computer program logic may comprise: acquiring plurality of SMBG data points; classifying said SMBG data points within periods of time with predetermined durations; evaluating glucose values in each period of time; and indicating risk of hypoglycemia for a subsequent period of time based on said evaluation.

An aspect of an embodiment of the present invention provides a method for identifying and/or predicting patterns of high glucose variability of a user. The method may compromise: acquiring plurality of SMBG data points; classifying said SMBG data points within periods of time with predetermined durations; evaluating blood glucose variability in each said period of time; and indicating risk of higher variability for a subsequent period of time based on said evaluation.

An aspect of an embodiment of the present invention provides a system for identifying and/or predicting patterns of high glucose variability of a user, wherein the system comprises an acquisition module acquiring a plurality of blood glucose data points and a processor. The processor may be programmed to: classifying said SMBG data points within periods of time with predetermined durations; evaluating blood glucose variability in each said period of time; and indicating risk of higher variability for a subsequent period of time based on said evaluation.

An aspect of an embodiment of the present invention provides a computer program product comprising a computer useable medium having computer program logic for enabling at least one processor in a computer system to identify and/or predict patterns of high glucose variability of a user. The computer program logic may comprise: acquiring plurality of SMBG data points; classifying said SMBG data points within periods of time with predetermined durations; evaluating blood glucose variability in each said period of time; and indicating risk of higher variability for a subsequent period of time based on said evaluation.

An aspect of an embodiment of the present invention provides a method for identifying and/or predicting patterns of ineffective testing of a user. The method may compromise: acquiring plurality of SMBG data points; classifying said SMBG data points within periods of time with predetermined durations; calculating the percentage of SMBG readings in each said period of time; comparing percentage against preset thresholds; and indicating ineffective testing for said period of time.

An aspect of an embodiment of the present invention provides a system for identifying and/or predicting patterns of ineffective testing of a user, wherein the system comprises an acquisition module acquiring a plurality of blood glucose data points and a processor. The processor may be programmed to: classify said SMBG data points within periods of time with predetermined durations; calculate the percentage of SMBG readings in each said period of time; compare percentage against preset thresholds; and indicate ineffective testing for said period of time.

An aspect of an embodiment of the present invention provides a computer program product comprising a computer useable medium having computer program logic for enabling at least one processor in a computer system to identify and/or predict patterns of ineffective testing of a user. The computer program logic may comprise: acquiring plurality of SMBG data points; classifying said SMBG data points within periods of time with predetermined durations; calculating the percentage of SMBG readings in each said period of time; comparing percentage against preset thresholds; and indicating ineffective testing for said period of time.

These and other advantages and features of the invention disclosed herein, will be made more apparent from the description, drawings and claims that follow.

## BRIEF SUMMARY OF THE DRAWINGS

The accompanying drawings, which are incorporated into and form a part of the instant specification, illustrate several aspects and embodiments of the present invention and, together with the description herein, and serve to explain the principles of the invention. The drawings are provided only for the purpose of illustrating select embodiments of the invention and are not to be construed as limiting the invention

**Figure 1**: Graphical representation of the principal feedback loops of diabetes control;
**Figure 2**: Graphical representation of the Stochastic Model of Self-Regulation Behavior;
**Figure 3**: Graphical representation of the Concept of Real-Time Meter Messaging System, illustrating the meter's computation of characteristics of the testing patterns for the next time period and issuing a message using one of the algorithms presented in this disclosure following an SMBG reading.

**Figure 4**: Graphical representation of computation identifying temporal patterns of hyperglycemia, illustrated by the identification of the overnight period as a period of high risk for hyperglycemia when the BG threshold parameter is set at 200 mg/dl and the composite probability threshold is set at 0.6;

**Figure 5**: Graphical representation of computation identifying temporal patterns of hyperglycemia, illustrated by the identification of two time periods, 3-7 PM and 7-11 PM as periods of high risk for hyperglycemia when the BG threshold parameter is set at 200 mg/dl and the composite probability threshold is set at 0.6;

**Figure 6**: Graphical representation of the computation identifying temporal patterns of hypoglycemia, illustrated by the identification of the time period from 11 PM to 11 AM as a period of high risk for hypoglycemia when the BG threshold parameter is set at 70 mg/dl and the composite probability threshold is set at 0.1;

**Figure 7**: Graphical representation of the computation identifying patterns of increased variability, illustrated by the identification of 3-7 PM as a period of high variability when ADRR threshold is set at 40 and the probability threshold is set at 0.6;

**Figure 8**: Graphical representation of the computation identifying ineffective SMBG testing patterns, illustrated by Subject A who only had 3.2% of all SMBG readings taken during the night [11PM-7AM) and Subject B who only had 4% of all SMBG readings taken in the afternoon (3-7 PM).

**Figure 9**: Functional block diagram for a computer system for implementation of embodiments of the present invention;

**Figure 10**: Schematic block diagram for an alternative variation of an embodiment of the present invention relating processors, communications links, and systems;

**Figure 11**: Schematic block diagram for another alternative variation of an embodiment of the present invention relating processors, communications links, and systems;

**Figure 12**: Schematic block diagram for a third alternative variation of an embodiment of the present invention relating processors, communications links, and systems.

## DETAILED DESCRIPTION OF THE INVENTION

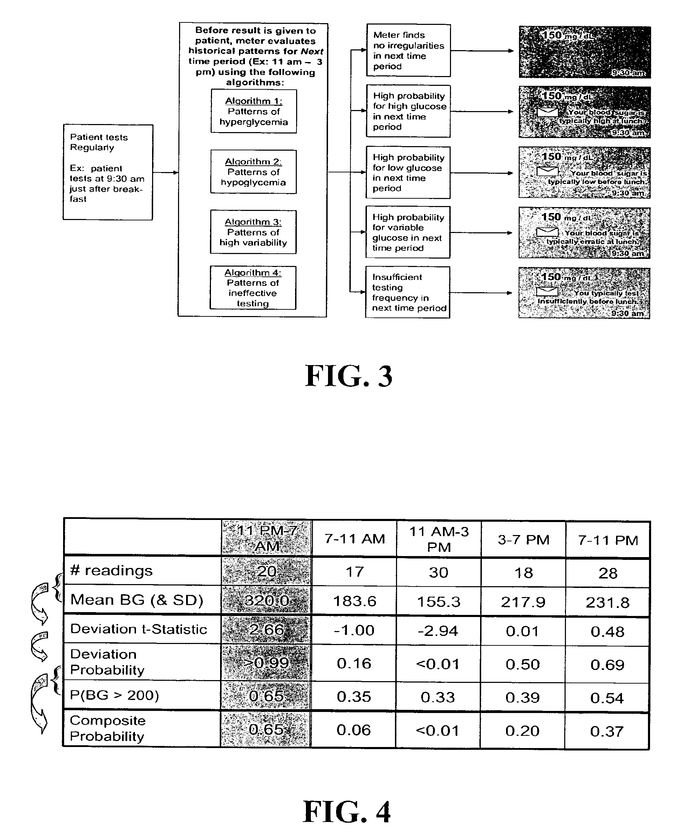

An aspect of various embodiments of this invention is, but not limited thereto, that providing real-time information to the patient about upcoming periods of possible hyperglycemia, possible hypoglycemia, increased glucose variability, or insufficient or excessive testing, will prompt appropriate treatment reaction and will thereby lead to better diabetes control. **Figure 3** illustrates this basic concept:

At each SMBG measurement and prior to the presentation of SMBG result the device evaluates historical patterns of glycemia and, based on this evaluation, issues warnings for the next time period. These warning include high risk for hyperglycemia or hypoglycemia, increased glucose variability, insufficient or excessive testing (**Figure 3**).

The systems, methods and algorithms enabling the messaging system are the subject of this invention disclosure. The theoretical background has been established by our theory of risk analysis of BG data (27, 28, 30) and follows previously developed and disclosed technology. All methods and algorithms have been first developed using general statistical assumptions for the deviation of glucose levels or testing patterns within a certain time period from the grand glucose mean or optimal pattern of a person. Then, the resulting algorithms were applied to a large data set (N=335 subjects) to validate the algorithms and to determine ranges for the algorithm parameters. **Table 1** presents demographic characteristics of the participants in this data set:

**Table 1**: Demographic characteristics and SMBG frequency in the validation data set:

| | |
|---|---|
| Age distribution: < 20, 20-40, > 40 years | 24.5%, 22.4%, 48.4%* |
| Gender: % Male vs. Female | 39% vs. 56.1%* |
| Race: White, African American, Hispanic, Native American, Asian, Other or missing | 76.7%, 12.8%, 4.2%, 0.6%, 0.3%, 5.4% |
| Type of diabetes: % T1DM vs. T2DM | 75.8% vs. 24.2% |
| Baseline HbA$_{1c}$ (SD) | 8.1 (1.3) |
| Average number of SMBG readings per day during the study (SD) | 4.1 (1.8) |
| * For these characteristics there were missing data, which results in percentages not adding up to 100%: | |

Duration:

The predetermined duration of the next time period covered by the warning message could be anywhere between 2 and 8 hours, preferably 6 hours. A day, i.e. a twenty-four hour period, may be divided into time bins with predetermined durations. For simplicity of the description, throughout this disclosure we will assume time periods with a predetermined duration of 4-hour time periods, with an 8-hour time period during the night.

The next time period covered by the warning message can begin at any time, after any SMBG reading. For simplicity of the description, throughout this disclosure we assume that an SMBG reading is taken at 11 PM, which initializes the next time period of a predetermined duration, e.g. 11 PM-7 AM.

An aspect of the present invention method includes providing indications of the risk of hyperglycemia, risk of hypoglycemia, risk of high glucose variability, and ineffective testing of a user for a next period of time, i.e. a subsequent period of time, based on the evaluation of glucose values in each period of time. The indication of the risks or ineffective testing may occur after the completion of the following steps: the acquisition of plurality of SMBG data points, the classification of SMBG data points within periods of time with predetermined durations, and the evaluation of glucose values in each period of time. The indications may be in the form of messages that are issued to the user indicating risk or ineffective testing immediately prior to the next period of time. Indications may occur during, but are not limited to, the following times: immediately prior to the next period of time, within 24 hours of acquisition of a plurality of SMBG data points, within 12 hours of acquisition of plurality of SMBG data points, within 6 hours of acquisition of a plurality of SMBG data points, near contemporaneously to the latest SMBG testing, and occurring in real time as well.

Number of readings:

The number of weeks of SMBG readings may be from about 2 weeks or over 6 weeks, but is preferably around 2 to 6 weeks, specifically about 4 weeks. Preferably, there are five readings per time period. The total number of SMBG readings may be from at least 30 readings, but preferably 60.

## 1. Algorithms Identifying Patterns of Hyperglycemia and Hypoglycemia from SMBG Data

The algorithms identifying patterns of increased risk for hyperglycemia and hypoglycemia work through several sequential steps described in detail below. The idea is that a 24-hour daily SMBG profile of a person is split into fixed periods of time with a predetermined duration, beginning at the time of a SMBG reading, or at another predetermined time. Then, based on historical SMBG data, the average glucose in each period of time is evaluated for deviations towards hyperglycemia or hypoglycemia. These deviations are assessed at two levels for:

■ Exceeding absolute thresholds identified by population data, literature, and accepted clinical practice guidelines, and

■ Exceeding idiosyncratic threshold, i.e. individual threshold, determined via analysis of the glycemic patterns of each individual.

If any of these two conditions are present, the time period is declared as a period of high risk for hyperglycemia or hypoglycemia. The judgment of each of the conditions is governed by a pair of parameters for hyperglycemia and a pair of parameters for hypoglycemia, as follows:

For risk of hyperglycemia:

■ BG threshold parameter ($\alpha 1$), reflecting population-level definition of high BG. For example, $\alpha 1$=180 mg/dl or $\alpha 1$=200 mg/dl are acceptable values.

■ Individual composite probability threshold ($\beta 1$) reflecting the idiosyncratic chance that BG would be high for this particular individual during this particular period of time. For example, $\beta 1$= 0.4 to 0.6 are acceptable values.

For risk of hypoglycemia:

■ BG threshold parameter ($\alpha 2$), reflecting population-level definition of low BG. For example, $\alpha 2$=70 mg/dl or $\alpha 2$=75 mg/dl are acceptable values.

■ Individual composite probability threshold (β2) reflecting the idiosyncratic chance that BG would be low for this particular individual during this particular period of time. For example, β = 0.01 to 0.2 are acceptable values.

Steps of the Algorithms Identifying Patterns of Hyperglycemia and Hypoglycemia: The first seven steps of these two algorithms are identical:

(1) Retrieve all SMBG data collected during the last 2-6 weeks of monitoring together with the time of each reading;

(2) At the time of SMBG reading, split each 24-hour day into M time bins with predetermined duration (2-8 hours). The time bins are defined as periods of sufficient duration allowing the accumulation of sufficient number of SMBG readings over 2-6 weeks. For example, assuming an SMBG reading at 11 PM, $M=5$ time bins can be defined as follows: 1~[11 PM-7AM); 2~[7-11 AM); 3~[11 AM - 3 PM); 4~[3-7 PM), 5~[7-11 PM), where "]" means inclusive;

(3) Classify all SMBG readings into these time bins: Let $X_{k1}, X_{k2}, ....., X_{kNk}$ are the SMBG readings that fell into time bin k over the last 30 days of SMBG, e.g. $k=1,2, ...,M$; where $N_k$ = number of SMBG readings in bin $k$.

(4) For each time bin k compute average and SD of BG as follows:

$$\overline{X}_k = \frac{1}{N_k}\sum_{i=1}^{N_k} X_{ki} \; ; \; SD_k^2 = \frac{1}{N_k-1}\sum_{i=1}^{N_k}(X_{ki} - \overline{X}_k)^2$$

(5) Compute the pooled mean and standard deviation of all SMBG readings as follows:

$$\overline{X} = \frac{1}{N}\sum_{k=1}^{M}\sum_{i=1}^{N} X_{ki} \; ; \quad SD^2 = \frac{1}{(N-1)}\sum_{k=1}^{M}\sum_{i=1}^{N}(X_{ki} - \overline{X})^2 \text{, where } N = N_1 + ... + N_M$$

is the total number of SMBG readings;

(6) For each time bin $k$ compute a deviation contrast: $t_k = \dfrac{X_k - \overline{X}}{\sqrt{\dfrac{SD^2}{N} + \dfrac{SD_k^2}{N_k}}}$. Alternatively, a deviation contrast can be computed using the grand mean via the formula $t_k = \sqrt{N}\dfrac{X_k - Y_k}{SD1}$, where Yk is the average of the means in the 4 time bins other than $k$ and $SD1$ is an estimate of the SD of $X_k$-$Y_k$. For example, for $_{k=2}$ $Y_2 = \frac{1}{4}(X_1 + X_3 + X_4 + X_5)$

Given the null hypothesis that the mean in time bin k is not higher than the means in the other time bins, the statistic $t_k$ will have a close to $t$-distribution, which for $N>30$ can be approximated by a central normal distribution. In the validation data set the average absolute error of this approximation was 0.0009 (SD=0.001), thus the normal approximation is acceptable for the practical implementation of the algorithms. (NOTE: computing directly a t-distribution is quite difficult, which is the reason for the recommended normal approximation). The normal approximation of the probability that $t_k > 0$ can be computed as P $(t_k > 0) = \Phi(t_k)$, where $\Phi(t_k)$ is the distribution function of a central normal distribution (with mean zero and SD=1).

(7) $\Phi(t_k)$ is approximated by a polynomial using the following code with z = $t_k$:

```
static double NormalCDF(double z)
    {
        if (z > 6) return 1.0;
        if (z < -6) return 0.0;
```

```
double b1 = 0.31938153;
double b2 = -0.356563782;
double b3 = 1.781477937;
double b4 = -1.821255978;
double b5 = 1.330274429;
double p = .2316419;
double c2 = 0.3989423;
double a = Math.Abs(z);
double t = 1.0 / (1.0 + a * p);
double b = c2 * Math.Exp(-z * z / 2);
double Φ = ((((b5*t+b4) * t+b3) * t+b2) * t+b1) * t;
Φ = 1.0 - b * CDF ;
if (z < 0) Φ = 1.0 - Φ ;
return n;
}
```

Note: This and other approximations of the central normal cumulative distribution function (*CDF*) are available in the pubic domain.

For hyperglycemia:

a. Compute the probability of BG exceeding a certain preset threshold *al* (e.g. $\alpha 1$ = 180 mg/dl) computed as:

$$P_k(\alpha 1) = \frac{1}{N_k} \sum_{i=1}^{N_k} I_{ki}(\alpha 1) \text{, where}$$

$$I_{ki}(\alpha 1) = \begin{cases} 1, if & X_{ki} > \alpha 1 \\ 0, if & X_{ki} \le \alpha 1 \end{cases}$$

b. Compute the individual composite probability the average BG in a time bin k to exceed the preset threshold $\alpha 1$ (e.g. $\alpha 1$ = 180 mg/dl) <u>and</u> the mean BG in time bin k to be higher than the rest of the bins (or than the grand mean):

$$CP_k(\alpha 1) = P_k(\alpha 1).\Phi(t_k);$$

c. If the composite probability $CP_k(\alpha 1)$ exceeds certain threshold $\beta 1$ (e.g. $\beta 1$ = 0.5), identify the time slot k as a period of increased risk for hyperglycemia and issue a message.

For hypoglycemia:

(8b) Compute the probability of BG to be lower than a certain threshold $\alpha 2$ (e.g. $\alpha 2$ = 70 mg/dl):

$$P_k(\alpha 2) = \frac{1}{N_k} \sum_{i=1}^{N_k} I_{ki}(\alpha 2) \text{, where}$$

$$I_{ki}(\alpha2) = \begin{cases} 1, if & X_{ki} < \alpha2 \\ 0, if & X_{ki} \geq \alpha2 \end{cases}$$

(9b) Compute the individual composite probability the average BG in a time bin k to be lower than the threshold $\alpha2$ (e.g. $\alpha2$ = 70 mg/dl) and the mean BG in time bin k to be lower than the rest of the bins (or than the grand mean):

$$CP_k(\alpha2) = P_k(\alpha2).(1-\Phi(t_k));$$

(10b) If the composite probability $CP_k(\alpha2)$ exceeds certain threshold $\beta2$ (e.g. $\beta2$ = 0.1), identify the time slot k as a period of increased risk for hypoglycemia and issue a message.

Thresholds of the Algorithms: The specific threshold values $\alpha i$ and $\beta I$ (i=1,2) used by the algorithms should be determined by the manufacturer of the device using the algorithms, the clinician managing the patient using the algorithms, or the user, and should be based on the acceptability of the frequency of messages vs. utility of the messages. Using the database presented in the beginning of this section, we have compiled Table 2, which presents the frequency of the messages issued by the algorithm for hyperglycemia and Table 3, which presents the frequency of the messages issued by the algorithm for hypoglycemia, given various thresholds. At least 60 SMBG readings over 30 days and at least 5 readings per time bin were required for a subject to enter this computation.

**Table 2:** Frequency of messages (percent of subjects who would get a message) identifying hyperglycemia within at least one of the time slots identified in #2 above:

| Population-based BG Threshold ($\alpha1$) | Threshold for Individual Composite Probability ($\beta1$) | | | | |
|---|---|---|---|---|---|
| | 0.3 | 0.4 | 0.5 | 0.6 | 0.7 |
| 180 mg/dl | 88.2% | 76.5% | 65.6% | 50.7% | 33.9% |
| 200 mg/dl | 81.4% | 67.4% | 53.4% | 35.3% | 21.7% |
| 225 mg/dl | 67.9% | 51.6% | 36.2% | 24.4% | 14.0% |
| 250 mg/dl | 55.7% | 40.7% | 26.2% | 13.6% | 8.1% |

**Table 3:** Frequency of messages (percent of subjects who would get a message) identifying hypoglycemia within at least one of the time slots identified in #2 above:

| Population-based BG Threshold (a2) | Threshold for Individual Composite Probability ($\beta2$) | | | | |
|---|---|---|---|---|---|
| | 0.05 | 0.1 | 0.15 | 0.2 | 0.25 |
| 65 mg/dl | 70.6% | 51.6% | 38.5% | 25.8% | 14.9% |
| 70 mg/dl | 75.1% | 60.2% | 47.1% | 33.5% | 21.7% |
| 75 mg/dl | 81.4% | 70.6% | 56.1% | 39.4% | 27.6% |
| 80 mg/dl | 84.6% | 75.6% | 64.3% | 48.9% | 35.3% |

The percentages in **Tables 2 and 3** were computed as follows: For each subject and for each time bin we computed whether a message would be "issued." If at least one message was "issued" for a subject, this subject was counted as 1 toward the frequency count in the tables.

Illustrative Examples:

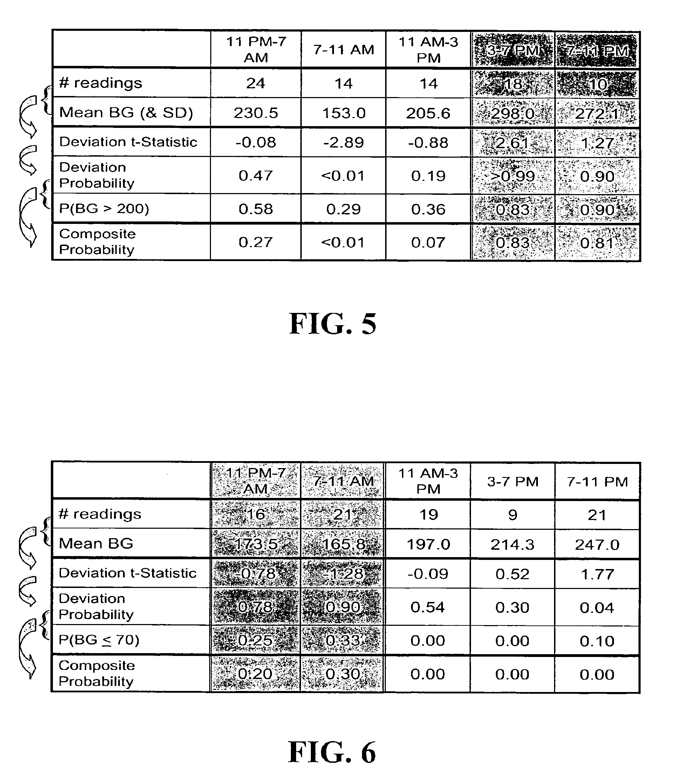

**Figures 4-6** illustrate the computation identifying temporal patterns of hyperglycemia. In **Figure 4**, the overnight period is identified as high risk for hyperglycema. In **Figure 5**, two time periods, 3-7 PM and 7-11 PM are identified as high risk for hyperglycemia. In **Figure 6**, the time period from 11 PM to 11 AM is identified as high risk for hypoglycemia.

## 2. Algorithm Identifying Patterns of Increased Glucose Variability from SMBG Data

The logic of the algorithm identifying patterns of increased glucose variability is similar to the logic of the algorithm identifying patterns of hyperglycemia. Instead of average BG, however, the test includes a measure of variability in each time bin. For example, such a measure could be the standard deviation (SD) of BG, or the risk standard deviation (RSD) of these values converted into risk space (28). In this implementation, we use the RSD because this measure is equally sensitive to hypoglycemic and hyperglycemic glucose variability.

The overall variability of a person is computed using the ADRR (average daily risk range), but can be also computed using the overall standard deviation of SMBG readings, M-value (37), MAGE (38), Lability Index (35), or any other accepted measure of variability (see Appendix A, 30, for a comprehensive list of possibilities). The standard deviation of SMBG readings would make the variability profile more sensitive to hyperglycemic excursions and less sensitive to hypoglycemia. In this implementation, we use the ADRR because this measure of variability has been shown to be superior in terms of its sensitivity to, and predictive ability of extreme glycemic excursions, and because it has clearly identified population thresholds (Appendix A, 30).

As in the previous section, it is assumed that an SMBG reading is taken at 11 PM and the subsequent 24-hour time period is split into time bins with a predetermined duration. Then, based on historical SMBG data, the glucose readings in each time bin are evaluated for deviations towards higher variability. These deviations are assessed for exceeding idiosyncratic threshold determined via analysis of the glycemic patterns of each individual. In addition, the overall ADRR of a person is classified with respect to population parameters. The combination of idiosyncratic deviations and overall ADRR is used to declare time period as high risk for increased variability. The judgment of each of the conditions is governed by two parameters:

■ ADRR threshold parameter ($\alpha$), reflecting population-level definition of high glucose variability. For example, $\alpha$=30, or $\alpha$=40 mg/dl are acceptable values.
■ Individual probability threshold ($\beta$) reflecting the idiosyncratic chance that variability would be high for this particular individual during this particular period of time. For example, $\beta$ = 0.6 to 0.8 are acceptable values.

Steps of the Algorithm:

(1) Retrieve all SMBG data collected during the last 2-6 weeks of monitoring together with the time of each reading;

(2) At the time of SMBG reading, split each 24-hour day into M time bins with predetermined duration (2-8 hours). The time bins are defined as periods of sufficient duration allowing the accumulation of sufficient number of SMBG readings over 2-6 weeks. For example, if a reading is taken at 11 PM, $M=5$ time bins can be defined as follows: 1~ (11 PM-7AM]; 2~(7-11 AM]; 3~(11 AM - 3 PM]; 4~(3-7 PM], 5~(7-11 PM], where "]" means inclusive;

(3) Classify all SMBG readings into these time bins: Let $X_{k1}$, $X_{k2}$, ....., $X_{kNk}$ are the SMBG readings that fell into time bin k over the last 30 days of SMBG, e.g. $k=1,2, ...,M;$ where $N_k$ = number of SMBG readings in bin k.

(4) Transform each BG reading into "risk space" using the previously introduced formula: $f(BG,a,b) = c. [(ln (BG))^a-b\}]$, where the parameters of this function depend on the BG scale and are as follows: If BG is measured in mg/dl, then $a=1.084, b=5.381, c=1.509.$ If BG is measured in mmol/l, then $a=1.026, b=1.861$ and $c=1.794$ (28).

(5) For each time bin, compute the risk standard deviation, RSD, using the formula:

$$RSD_k^2 = \frac{1}{N_k - 1}\sum_{i=1}^{N_k}(f(X_{ki}) - f\overline{X}_k)^2, \text{ where } f\overline{X}_k = \frac{1}{N_k}\sum_{i=1}^{N_k}fX_{ki}.$$

(6) Compute the overall *RSD* using the formulas above, but including all SMBG readings across all time bins.

(7) For each time bin compute the ratios $Z_k=5^*(RSD_k/RSD\text{-}1)$. As shown by the analysis of large database (N=233 subjects), these ratios have approximately central normal distribution and therefore can be used for testing of idiosyncratic deviations in the same way as in the previous section.

(8) For each time bin, compute each individual's probability that $Z_k > 0$ as $P (Z_k > 0) = \Phi(Z_k)$, where $\Phi$ is the distribution function of central normal distribution computed by the polynomial approximation given in the previous section. $P (Z_k > 0)$ is the individual probability that a certain time bin will have higher variability than the others.

(9) Compute the ADRR of each person as an overall marker of variability, using the previously disclosed, December 12, 2006, and recently published (30) algorithm. In brief, the computation of the ADRR is accomplished by the following formulas:

For each SMBG reading compute $r(BG)=10.f(BG)^2$, where f(BG) is defined in (4) above;

Compute *rl(BG)=r(BG)* if *f(BG)<0* and *0* otherwise;

Compute *rh(BG)=r(BG)* if *f(BG)>0* and *0* otherwise.

Let $x_1^1, x_2^1, ... x_n^1$ be a series of $n^1$ SMBG readings taken on Day 1;

....

Let $x_1^M, x_2^M, ... x_n^M$ *be* a series of $n^M$ SMBG readings taken on Day M.

Where $n^1, n^2, ...., n^M \geq 3$ and the number of days of observation M is between 14 and 42;

$LR^i = \max (rl(x_1^i), rl(x^i_2), ..., rl(x^i_n))$ and

$HR^i = \max (rh(x_1^i), rh(x_2^i), ... , rh(x_n^i))$ for day # *i; i = 1, 2, ... M.*

The Average Daily Risk Range is then defined as:

$$ADRR \;\; = \;\; \frac{1}{M} \sum_{i=1}^{M} [\, LR^{\,i} + HR^{\,i} \,] .$$

Thresholds of the Algorithm: The specific threshold values $\alpha$ and $\beta$ used by the algorithm should be determined by the manufacturer of the device using the algorithms based on the acceptability of the frequency of messages vs. utility of the messages. Using the database described above we have compiled **Table 4,** which presents the frequency of the messages issued by the algorithm, given various thresholds. At least 60 SMBG readings over 30 days and at least 5 readings per time bin were required for a subject to enter this computation.

**Table 4:** Frequency of messages (percent of subjects who would get a message) identifying higher glucose variability within a certain time bin:

| | Threshold for Individual Probability ($\beta$) | | | | |
|---|---|---|---|---|---|
| **Population-based ADRR Threshold ($\alpha$)** | **0.5** | **0.6** | **0.7** | **0.8** | **0.9** |
| **ADRR > 20** | 77.7% | 71.7% | 55.4% | 33.5% | 15.9% |
| **ADRR > 30** | 59.7% | 54.5% | 40.8% | 25.3% | 12.0% |
| **ADRR > 40** | 29.6% | 26.2% | 19.7% | 9.4% | 5.2% |
| **ADRR > 50** | 10.7% | 9.0% | 7.3% | 3.9% | 1.3% |

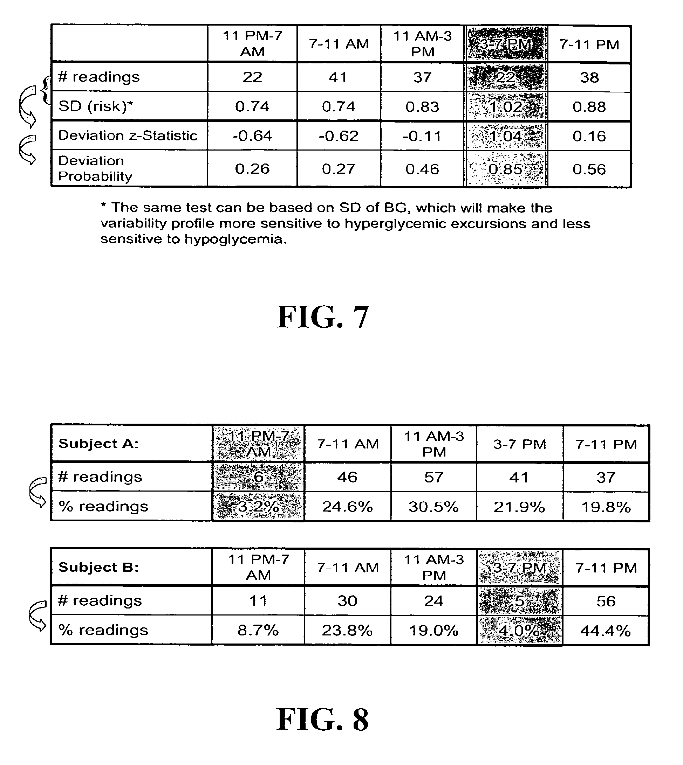

<u>Illustrative Example:</u> **Figure 7** illustrates the computation identifying patterns of increased variability: The period 3-7 PM is identified as a period of high variability; The ADRR threshold is set at 40; the probability threshold is set at 0.6.

### 3. Algorithm Identifying Ineffective SMBG Testing Patterns

Similarly to the previously described algorithms, we assume that an SMBG reading is taken at 11 PM and then the algorithm identifying ineffective testing patterns splits the day into five time bins: 1~[11 PM-7AM); 2~[7-11 AM); 3~[11 AM - 3 PM); 4~[3-7 PM), and 5~[7-11 PM)). Then, the algorithm computes the percentage of SMBG readings contained in each time bin.

If a time bin is found contains less than $\alpha$%, or more than $\beta$% of a person's SMBG readings, then this time bin will be identified as a period of insufficient, or excessive testing, respectively. The parameters $\alpha$% and $\beta$% can be set at any reasonable values, e.g. $\alpha$% = 5% and $\beta$% = 50%. If one or both of these thresholds is exceeded, a message would be issued as presented in **Figure 3.** The message would include a warning about insufficient testing if the testing frequency is below $\alpha$%, and a warning about excessive testing is the testing frequency is higher than $\beta$%.

<u>Illustrative Example:</u> **Figure 8** illustrates ineffective SMBG testing patterns. With a threshold for insufficient testing set at 5%, Subject A had only 3.2% of his/her SMBG readings during the night, while Subject B had only 4% SMBG readings in the afternoon.

In the database used for validation of these methods, there were no examples of excessive sampling within a time bin for $\beta$% = 50%. The highest testing frequency of all subjects across all time bins was 48.4% in the morning time bin (7-11 AM).

<u>Exemplary Systems:</u>

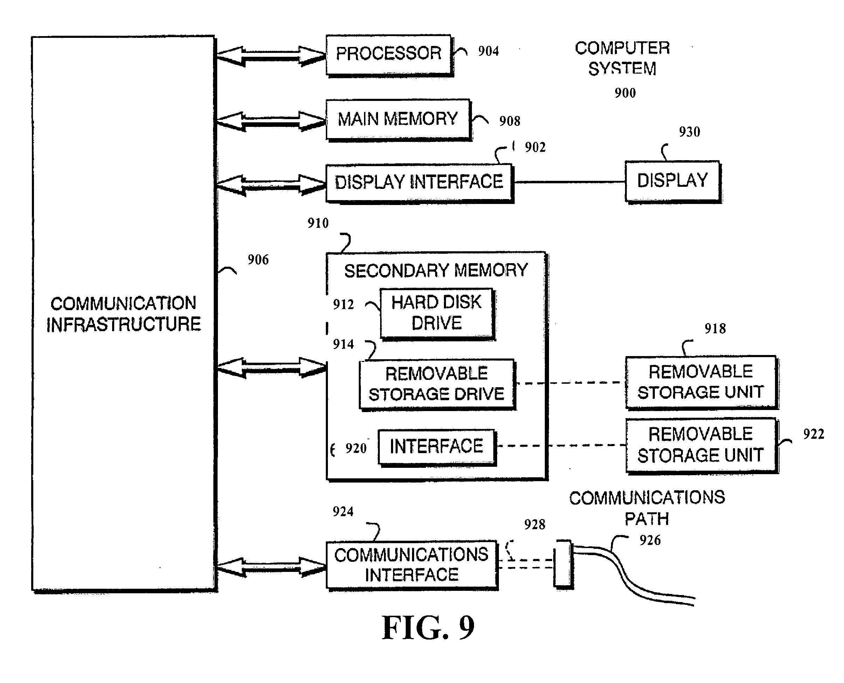

The method of the invention may be implemented using hardware, software or a combination thereof and may be implemented in one or more computer systems or other processing systems, such as a personal digital assistance (PDAs), or directly in blood glucose self-monitoring devices (e.g. SMBG memory meters) equipped with adequate memory and processing capabilities. In an example embodiment, the invention may be implemented in software running on a general purpose computer **900** as illustrated in **Figure 9.** Computer system **900** may include one or more processors, such as processor **904.** Processor **904** may be connected to a communications infrastructure **906** (e.g. a communications bus, cross-over bar, or network). Computer system **900** may include a display interface **902** that forwards graphics, text, or other data from the communications infrastructure **906** (or from a frame buffer not shown) for display on the display unit **930.** Display unit **930** may be digital and/or analog.

Computer system **900** may also include a main memory **908,** preferably random access memory (RAM), and may also include a secondary memory **910.** The secondary memory **910** may include, for example, a hard disk drive **912** and/or a removable storage drive **914,** representing a floppy disk drive, a magnetic tape drive, an optical disk drive, a flash memory, etc. The removable storage drive **914** reads from and/or writes to a removable storage unit **918** in a well known manner. Removable storage unit **918,** represents a floppy disk, magnetic tape, optical disc, etc. which is read by and written to by removable storage drive **914.** As will be appreciated, the removable storage unit **918** may include a computer usable storage medium having stored therein computer software and/or data.

In alternative embodiments, secondary memory **910** may include other means for allowing computer programs or other instructions to be loaded into computer system **900.** Such means may include, for example, a removable storage unit **922** and an interface **920.** Examples of such removable storage units/interfaces include a program cartridge and cartridge interface (such as that found in video game devices), a removable memory chip (such as a ROM, PROM, EPROM or EEPROM) and associated socket, and other removable storage units **922** and interfaces **920** which allow software and data to be transferred from the removable storage unit **922** to computer system **900.**

Computer system **900** may also include a communications interface **924.** Communications interface **924** allows software and data to be transferred between computer system **900** and external devices. Examples of communications interface **924** may include a modem, a network interface (such as an Ethernet card), a communications port (e.g., serial or parallel, etc.), a PCMCIA slot and card, etc. Software and data transferred via communications interface **924** may be in the form of signals **928** which may be electronic, electromagnetic, optical or other signals capable of being received by communications interface **924.** Signals **928** may be provided to communications interface **924** via a communications path (i.e., channel) **926.** Channel **926** carries signals **928** and may be implemented using wire or cable, fiber optics, a phone line, a cellular phone link, an RF link, an infrared link, and other communications channels.

In this document, the terms "computer program medium" and "computer usable medium" are used to generally refer to media such as removable storage drive **914,** a hard disk installed in hard disk drive **912,** and signals **928.** These computer program products are means for providing software to computer systems **900.** The invention includes such computer program products.

Computer programs (also called computer control logic) may be stored in main memory 908 and/or secondary memory **910.** Computer programs may also be received via communications interface **924.** Such computer programs, when executed, enable computer system **900** to perform the features of the present invention as discussed herein. In particular, the computer programs, when executed, enable processor **904** to perform the functions of the present invention. Accordingly, such computer programs represent controllers of computer system **900.**

In an embodiment where the invention is implemented using software, the software may be stored in a computer program product and loaded into computer system **900** using removable storage drive **914,** hard drive **912** or communications interface **924.** The control logic (software), when executed by the processor **904,** causes the processor **904** to perform the function of the invention as described herein.

In another embodiment, the invention is implemented primarily in hardware using, for example, hardware components such as application specific integrated circuits (ASICs). Implementation of the hardware state machine to perform the functions described herein will be apparent to persons skilled in the relevant art(s).

In yet another embodiment, the invention is implemented using a combination of both hardware and software.

In an example software embodiment of the invention, the methods described above may be implemented in SPSS control language, but could be implemented in other programs, such as, but not limited to, C++ program language or other programs available to those skilled in the art.

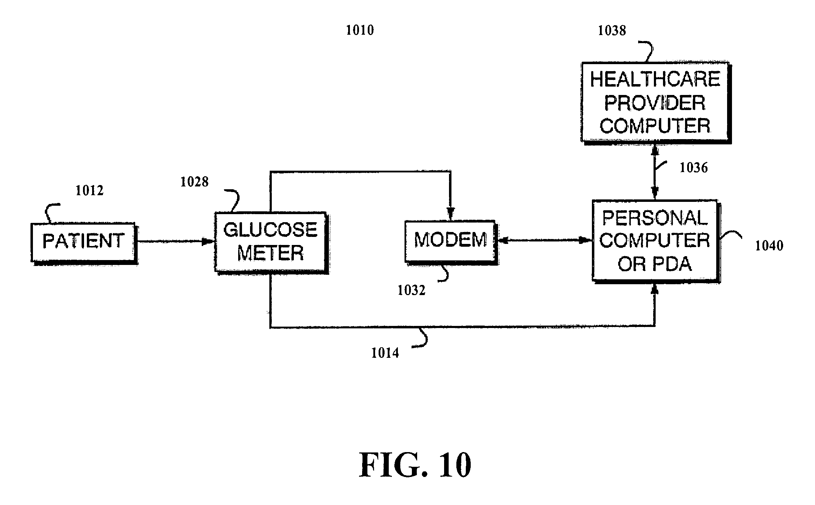

**Figures 10-12** show block diagrammatic representations of alternative embodiments of the invention. Referring to **Figure 10,** there is shown a block diagrammatic representation of the system **1010** essentially comprises the glucose meter **1028** used by a patient **1012** for recording, *inter alia,* insulin dosage readings and measured blood glucose ("BG") levels. Data obtained by the glucose meter **1028** is preferably transferred through appropriate communication links **1014** or data modem **1032** to a processor, processing station or chip **1040,** such as a personal computer, PDA, or cellular telephone, or via appropriate Internet portal. For instance data stored may be stored within the glucose meter **1028** and may be directly downloaded into the personal computer **1040** through an appropriate interface cable and then transmitted via the Internet to a processing location. An example is the ONE TOUCH monitoring system or meter by LifeScan, Inc. which is compatible with IN TOUCH software which includes an interface cable to download the data to a personal computer. It should be appreciated that the glucose meter 1028 and any of the computer processing modules or storage modules may be integral within a single housing or provided in separate housings.

The glucose meter is common in the industry and includes essentially any device that can function as a BG acquisition mechanism. The BG meter or acquisition mechanism, device, tool or system includes various conventional methods directed towards drawing a blood sample (e.g. by fingerprick) for each test, and a determination of the glucose level using an instrument that reads glucose concentrations by electromechanical methods. Recently, various methods for determining the concentration of blood analytes without drawing blood have been developed. For example, U.S. Pat. No. 5,267,152 to Yang et al. (hereby incorporated by reference) describes a noninvasive technique of measuring blood glucose concentration using near-IR radiation diffuse-reflection laser spectroscopy. Similar near-IR spectrometric devices are also described in U.S. Pat. No. 5,086,229 to Rosenthal et al. and U.S. Pat. No. 4,975,581 to Robinson et al. (of which are hereby incorporated by reference).

U.S. Pat. No. 5,139,023 to Stanley (hereby incorporated by reference) describes a transdermal blood glucose monitoring apparatus that relies on a permeability enhancer (e.g., a bile salt) to facilitate transdermal movement of glucose along a concentration gradient established between interstitial fluid and a receiving medium. U.S. Pat. No. 5,036,861 to Sembrowich (hereby incorporated by reference) describes a passive glucose monitor that collects perspiration through a skin patch, where a cholinergic agent is used to stimulate perspiration secretion from the eccrine sweat gland. Similar perspiration collection devices are described in U.S. Pat. No. 5.076,273 to Schoendorfer and U.S. Pat. No. 5,140,985 to Schroeder (of which are hereby incorporated by reference).

In addition, U.S. Pat. No. 5,279,543 to Glikfeld (hereby incorporated by reference) describes the use of iontophoresis to noninvasively sample a substance through skin into a receptacle on the skin surface. Glikfeld teaches that this sampling procedure can be coupled with a glucose-specific biosensor or glucose-specific electrodes in order to monitor blood glucose. Moreover, International Publication No. WO 96/00110 to Tamada (hereby incorporated by reference) describes an iotophoretic apparatus for transdermal monitoring of a target substance, wherein an iotophoretic electrode is used to move an analyte into a collection reservoir and a biosensor is used to detect the target analyte present in the reservoir. Finally, U.S. Pat. No. 6,144,869 to Berner (hereby incorporated by reference) describes a sampling system for measuring the concentration of an analyte present.

Further yet, the BG meter or acquisition mechanism may include indwelling catheters and subcutaneous tissue fluid sampling.

The computer, processor or PDA **1040** may include the software and hardware necessary to process, analyze and interpret the self-recorded diabetes patient data in accordance with predefined flow sequences and generate an appropriate data interpretation output. The results of the data analysis and interpretation performed upon the stored patient data by the computer **1040** may be displayed in the form of a paper report generated through a printer associated with the personal computer **940.** Alternatively, the results of the data interpretation procedure may be directly displayed on a video display unit associated with the computer **940.** The results additionally may be displayed on a digital or analog display device. Preferably, the results may be displayed according to the characteristics presented in **Figures 7** or **8.** The personal computer 1040 may transfer data to a healthcare provider computer **1038** through a communication network 1036. The data transferred through communications network **1036** may include the self-recorded diabetes patient data or the results of the data interpretation procedure.

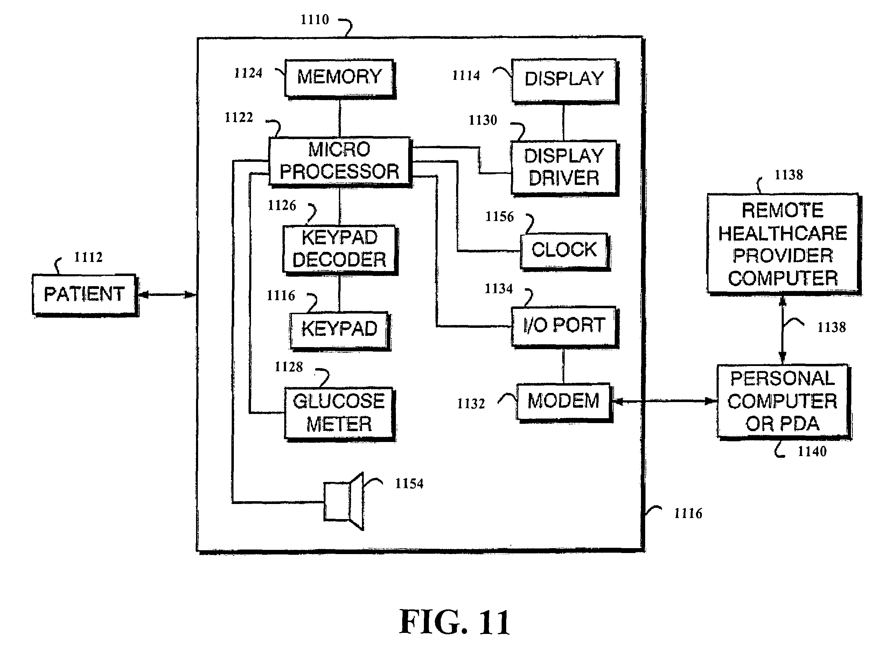

**Figure 11** shows a block diagrammatic representation of an alternative embodiment having a diabetes management system that is a patient-operated apparatus **1110** having a housing preferably sufficiently compact to enable apparatus **1110** to be hand-held and carried by a patient. A strip guide for receiving a blood glucose test strip (not shown) is located on a surface of housing **1116.** Test strip receives a blood sample from the patient **1112.** The apparatus may include a microprocessor **1122** and a memory **1124** connected to microprocessor **1122.** Microprocessor **1122** is designed to execute a computer program stored in memory **1124** to perform the various calculations and control functions as discussed in greater detail above. A keypad **1116** may be connected to microprocessor **1122** through a standard keypad decoder **1126.** Display **1114** may be connected to microprocessor **1122** through a display driver **1130.** Display **1114** may display the characteristics featured in **Figures 7** or **8.** Display **1114** may be digital and/or analog. Speaker **1154** and a clock **1156** also may be connected to microprocessor **1122.** Speaker **1154** operates under the control of microprocessor **1122** to emit audible tones alerting the patient to possible future hypoglycemic or hyperglycemic risks. Clock **1156** supplies the current date and time to microprocessor **1122.**

Memory **1124** also stores blood glucose values of the patient **1112,** the insulin dose values, the insulin types, and the parameters used by the microprocessor **1122** to calculate future blood glucose values, supplemental insulin doses, and carbohydrate supplements. Each blood glucose value and insulin dose value may be stored in memory **1124** with a corresponding date and time. Memory **1124** is preferably a non-volatile memory, such as an electrically erasable read only memory (EEPROM).

Apparatus **1110** may also include a blood glucose meter **1128** connected to microprocessor **1122.** Glucose meter **1128** may be designed to measure blood samples received on blood glucose test strips and to produce blood glucose values from measurements of the blood samples. As mentioned previously, such glucose meters are well known in the art. Glucose meter **1128** is preferably of the type which produces digital values which are output directly to microprocessor **1122.** Alternatively, blood glucose meter **1128** may be of the type which produces analog values. In this alternative embodiment, blood glucose meter 1128 is connected to microprocessor **1122** through an analog to digital converter (not shown).

Apparatus **1110** may further include an input/output port **1134,** preferably a serial port, which is connected to microprocessor **1122.** Port **1134** may be connected to a modem **1132** by an interface, preferably a standard RS232 interface. Modem **1132** is for establishing a communication link between apparatus **1110** and a personal computer **1140** or a healthcare provider computer **1138** through a communication network **1136.** Specific techniques for connecting electronic devices through connection cords are well known in the art. Another alternative example is "Bluetooth" technology communication.

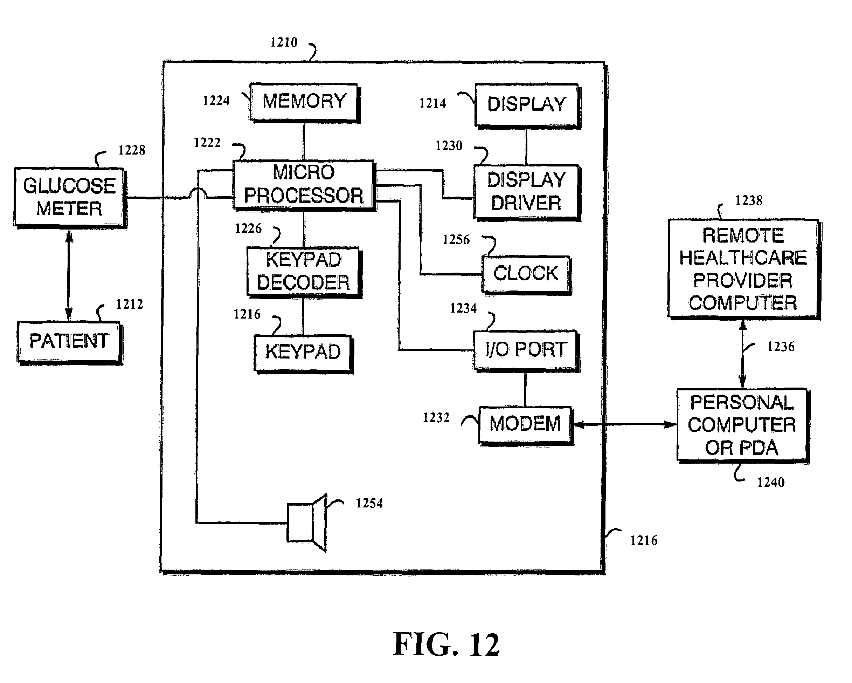

Alternatively, **Figure 12** shows a block diagrammatic representation of an alternative embodiment having a diabetes management system that is a patient-operated apparatus **1210,** similar to the apparatus as shown in **Figure 11,** having a housing preferably sufficiently compact to enable the apparatus **1210** to be hand-held and carried by a patient. For example, a separate or detachable glucose meter or BG acquisition mechanism/module **1228.** There are already self-monitoring devices that are capable of directly computing the algorithms disclosed in this application and displaying the results to the patient without transmitting the data to anything else. Examples of such devices are ULTRA SMART by LifeScan, Inc., Milpitas, CA and FREESTYLE TRACKER by Therasense, Alameda, CA.

Accordingly, the embodiments described herein are capable of being implemented over data communication networks such as the internet, making evaluations, estimates, and information accessible to any processor or computer at any remote location, as depicted in **Figures 9-12** and/or U.S. Pat. No. 5,851,186 to Wood, of which is hereby incorporated by reference herein. Alternatively, patients located at remote locations may have the BG data transmitted to a central healthcare provider or residence, or a different remote location.

It should be appreciated that any of the components/modules discussed in **Figures 9-12** may be integrally contained within one or more housings or separated and/or duplicated in different housings.

It should also be appreciated that any of the components/modules present in **Figures 9-12** may be in direct or indirect communication with any of the other components/modules.

In summary, the various embodiments of the invention propose a data analysis computerized (or non-computerized) method and system for the evaluation of the most important component of glycemic control in individuals with diabetes: glycemic variability. The method, while using only routine SMBG data, provides, among other things, an average daily risk range.

The potential implementations of the method, system, and computer program product of the various embodiments of the invention provide the following advantages, but are not limited thereto. First, the various embodiments of the invention enhance existing SMBG devices by introducing an intelligent data interpretation component capable of evaluating the effectiveness of SMBG testing by providing information about upcoming periods of possible hyperglycemia, possible hypoglycemia, increased glucose variability, and insufficient or excessive testing. They further enable future SMBG devices by the same features.

As an additional advantage, the various embodiments of the invention enhance hand-held devices (e.g. PDAs or any applicable devices or systems) intended to assist diabetes management.

Still yet another advantage, the various embodiments of the invention enhance software that retrieves SMBG data. This software can reside on patients' personal computers, or be used via Internet portal.

Moreover, the various embodiments of the invention may evaluate the effectiveness of various treatments for diabetes (e.g. insulin or variability lowering medications, such as pramlintide and exenatide).

Further still, the various embodiments of the invention may evaluate the effectiveness of new insulin delivery devices (e.g. insulin pumps), or of future closed-loop diabetes control systems.

Further yet, aspects of the present invention disclosure include, but not limited thereto, four methods and algorithms for identifying patterns of: (i) hyperglycemia, (ii) hypoglycemia, (iii) increased glucose variability, and (iv) ineffective self-testing during a time period subsequent to the time that the message is given. These algorithms use routine SMBG data collected over a period of 2-6 weeks and can be incorporated in self-monitoring devices, or software retrieving data from self-monitoring devices. All algorithms result in messages customized for the treatment pattern of a particular person, thereby providing base for customized idiosyncratic treatment.

The methods can be used separately, in combination, or in addition to previously described methods, to drive a system of messages delivered by the device to an individual with diabetes, in this case at a time proximal to a patient BG test. A theoretical model of self-regulation behavior asserts that such messages would be effective and would result in improved glycemic control.

REFERENCES

The following references are hereby incorporated by reference herein in their entirety:

1. Aaby Svendsen P, Lauritzen T, Soegard U, Nerup J. Glycosylated Haemoglobin and Steady-State Mean Blood Glucose Concentration in Type 1 (Insulin-Dependent) Diabetes. Diabetologia, 23: 403-405, 1982.

2. Bolli G: How to ameliorate the problem of hypoglycemia in intensive as well as nonintensive treatment of Type 1 diabetes. Diabetes Care, 22 (Supp. 2), B43-B52, 1999.

3. Clarke WL, Cox D, Gonder-Frederick LA, Carter W, Pohl SL. Evaluating the clinical accuracy of self-blood glucose monitoring systems. Diabetes Care, 10: 622-628, 1987.

4. Cox DJ, Gonder-Frederick LA, Polonsky W, Schlundt D, Julian D, Clarke WL: A multicenter evaluation of blood glucose awareness training-II. Diabetes Care, 18: 523-528, 1995.

5. Cox DJ, Gonder-Frederick LA, Polonsky W, Schlundt D, Julian D, Kovatchev BP, Clarke WL. Blood Glucose Awareness Training (BGAT-II): Long term benefits. Diabetes Care, 24: 637-642, 2001.

6. Cox DJ, Kovatchev B, Julian D, Gonder-Frederick LA, Polonsky WH, Schlundt DG, Clarke WL. Frequency of severe hypoglycemia in IDDM can be predicted from self-monitoring blood glucose data. Journal of Clinical Endocrinology and Metabolism 79: 1659-1662, 1994.

7. Cox DJ, Kovatchev BP, Clarke WL, Gonder-Frederick LA, Trajanoski Z. Accuracy of Home Blood Glucose Meters for Prediction of Severe Hypoglycemia (SH). Diabetes, 48, Supplement 1, A101, 1999.

8. Cranston I, Lomas J, Maran A, Macdonald I, Amiel S. Restoration of hypoglycemia unawareness in patients with long duration insulin-dependent diabetes mellitus. Lancet 344:283-287, 1994

9. Cryer PE. Hypoglycaemia: The limiting factor in the glycaemic management of type I and type II diabetes. Diabetologia 45: 937-948, 2002

10. Cryer PE. Iatrogenic hypoglycemia as a cause of hypoglycemia-associated autonomic failure in IDDM: A vicious cycle. Diabetes 41:255-260, 1992

11. Cryer PE: Hypoglycemia: The Limiting factor in the management of IDDM. Diabetes 43: 1378-1389, 1994

12. Dagogo-Jack S, Rattarasarn C, Cryer PE. Reversal of hypoglycemia unawareness, but not defective glucose counterregulation, in IDDM. Diabetes 43:1426-1434, 1994

13. Fanelli C, Epifano L, Rambotti A et al.: Meticulous prevention of hypoglycemia (near-) normalizes magnitude and glycemic thresholds of neuroendocrine responses to, symptoms of, and cognitive function during hypoglycemia in intensively treated patients with IDDM of short duration. Diabetes, 42, 1683-1689, 1993.

14. Fanelli CG, Pampanelli S, Epifano L et al. Longterm recovery from unawareness, deficient counterregulation and lack of cognitive dysfunction during hypoglycemia following institution of rational intensive therapy in IDDM. Diabetologia 37:1265-1276, 1994

15. Gold AE, Frier BM, MacLeod KM, Deary IJ. A structural equation model for predictors of severe hypoglycaemia in patients with insulin-dependent diabetes mellitus. Diabet Med, 14:309-315, 1997.

16. Gold AE, Deary IJ, Frier BM. Recurrent severe hypoglycaemia and cognitive function in type I diabetes. Diabet Med 10:503-508, 1993

17. Henderson JN, Allen KV, Deary IJ, Frier BM. Hypoglycemia in insulin-treated Type 2 diabetes: frequency, symptoms and impaired awareness. Diabet Med 20: 1016-1021, 2003

18. Inouye K, Shum K, Chan O, Mathoo JMR, Matthews SG, Vranic M. Effects of recurrent hyperinsulinemia with and without hypoglycemia on counterregulation in diabetic rats. Am JPhysiol Endocrinol Metab 282:E1369-E1379, 2002

19. Kinsley BT, Weinger K, Bajaj M, Levy CJ, Simonson DC, Quigley M, Cox DJ, Jacobson AM. Blood glucose awareness training and epinephrine responses to hypoglycemia during intensive treatment in type 1 diabetes. Diabetes Care, 22: 1022-1028, 1999.

20. Kovatchev BP, Cox DJ, Farhy LS, Straume M, Gonder-Frederick LA, Clarke, WL. Episodes of Severe Hypoglycemia in Type 1 Diabetes are Preceded, and Followed, within 48 Hours by Measurable Disturbances in Blood Glucose. J of Clinical Endocrinology and Metabolism, 85: 4287-4292, 2000.

21. Kovatchev BP, Cox DJ, Gonder-Frederick LA and WL Clarke. Methods for quantifying self-monitoring blood glucose profiles exemplified by an examination of blood glucose patterns in patients with Type 1 and Type 2 Diabetes. Diabetes Technol Ther, 4 (3): 295-303, 2002.

22. Kovatchev BP, Cox DJ, Gonder-Frederick LA and WL Clarke. Symmetrization of the blood glucose measurement scale and its applications. Diabetes Care 20: 1655-1658, 1997.

23. Kovatchev BP, Cox DJ, Gonder-Frederick LA Young-Hyman D, Schlundt D, Clarke WL. Assessment of risk for severe hypoglycemia among adults with IDDM: Validation of the Low Blood Glucose Index, Diabetes Care 21: 1870-1875, 1998.

24. Kovatchev BP, Cox DJ, Gonder-Frederick LA, Clarke WL. Low Blood Glucose Index Predicts Occurrence of Severe Hypoglycemia Among Adults with IDDM. Diabetes, 47, Supplement 1, A107, 1998.

25. Kovatchev BP, Cox DJ, Gonder-Frederick LA, Clarke WL. Transforming the Blood Glucose Scale: Statistical and Clinical implications. Diabetes, 46, Supplement 1, A268, 1997.

26. Kovatchev BP, Cox DJ, Gonder-Frederick LA, Schlundt D and WL Clarke. Stochastic model of self-regulation decision making exemplified by decisions concerning hypoglycemia. Health Psychology, 17:277-284, 1998.

27. Kovatchev BP, Cox DJ, Kumar A, Gonder-Frederick LA and WL Clarke. Algorithmic Evaluation of Metabolic Control and Risk of Severe Hypoglycemia in Type 1 and Type 2 Diabetes Using Self-Monitoring Blood Glucose (SMBG) Data. Diabetes Technology and Therapeutics, 5 (5): 817-828, 2003

28. Kovatchev BP, Straume M, Cox DJ, Farhy LS. Risk analysis of blood glucose data: A quantitative approach to optimizing the control of insulin dependent diabetes. J of Theoretical Medicine, 3:1-10, 2001.

29. Kovatchev BP, Cox DJ, Straume M, Farhy LS: Estimating the Speed of Blood Glucose Transitions and its Relationship with Severe Hypoglycemia. Diabetes, 48, Suppl. 1, A363, 1999.

30. Kovatchev BP, Otto E, Cox DJ, Gonder-Frederick LA, Clarke WL (2006). Evaluation of a New Measure of Blood Glucose Variability in Diabetes. Diabetes Care, 29: 2433-2438.

31. Lincoln NB, Faleiro RM, Kelly C, Kirk BA, Jeffcoate WJ. Effect of long-term glycemic control on cognitive function. Diabetes Care 19:656-658, 1996

32. Mathoo JMR, Shi ZQ, Klip A, Vranic M. Opposite effects of acute hypoglycemia and acute hyperglycemia on glucose transport and glucose transporters in perfused rat skeletal muscle. Diabetes 48: 1281-1288, 1999

33. McAulay V, Deary IJ, Frier BM: Symptoms of hypoglycaemia in people with diabetes. Diabet Med 18:690-705, 2001

34. Reichard P, Phil M. Mortality and treatment side effects during long-term intensified conventional insulin treatment in the Stockholm Diabetes Intervention study. Diabetes 43: 313-317, 1994

35. Ryan EA, Shandro T, Green K, Paty BW, Senior PA, Bigam D, Shapiro AMJ, Vantyghem MC. Diabetes 53: 955-962, 2004

36. Santiago JV. Lessons from the Diabetes Control and Complications Trial, Diabetes, 42:1549-1554, 1993.

37. Schlichtkrull J, Munck O, Jersild M. Acta Med Scand 177: 95-102, 1965

38. Service FJ , Molner GD, Rosevear JW, Ackerman E, Gatewood LC, Taylor WF. Diabetes 19: 644-655, 1970

39. The Diabetes Control and Complications Trial Research Group. Hypoglycemia in the Diabetes Control and Complications Trial. Diabetes 46: 271-286, 1997

40. The Diabetes Control and Complications Trial Research Group. The effect of intensive treatment of diabetes on the development and progression of long-term complications of insulin-dependent diabetes mellitus. N Engl J Med 329: 978-986, 1993

41. The diabetes research in children network (DirecNet) study group. A multicenter study of the accuracy of the One Touch® Ultra® home glucose meter in children with Type 1 diabetes. Diabetes Technol Ther, 5: 933-942, 2003

42. UK Prospective Diabetes Study Group (UKPDS). Intensive blood-glucose control with sulphonylureas or insulin compared with conventional treatment and risk of complications in patients with type 2 diabetes. Lancet 352: 837-853, 1998

43. Kovatchev BP, Cox DJ, Gonder-Frederick LA and WL Clarke (1997). Symmetization of the Blood Glucose Measurement Scale and Its Applications, Diabetes Care, 20, 1655-1658.

44. Kovatchev BP, Straume M, Cox DJ, Farhi LS (2001) Risk Analysis of Blood Glucose Data: A Quantitative Approach to Optimizing the Control of Insulin Dependent Diabetes. J of Theoretical Medicine, 3: 1-10.

45. Kovatchev BP, Cox DJ, Gonder-Frederick LA and WL Clarke (2002). Methods for quantifying self-monitoring blood glucose profiles exemplified by an examination of blood glucose patterns in patients with Type 1 and Type 2 Diabetes. Diabetes Technology and Therapeutics, 4 (3): 295-303.

46. Kovatchev BP, Cox DJ, Kumar A, Gonder-Frederick LA, Clarke WL (2003). Algorithmic Evaluation of Metabolic Control and Risk of Severe Hypoglycemia in Type 1 and Type 2 Diabetes Using Self-Monitoring Blood Glucose (SMBG) Data. Diabetes Technology and Therapeutics, 5 (5): 817-828.

47. Kovatchev BP, Otto E, Cox DJ, Gonder-Frederick LA, Clarke WL (2006). Evaluation of a New Measure of Blood Glucose Variability in Diabetes. Diabetes Care, 29: 2433-2438.

It should be appreciated that various aspects of embodiments of the present method, system, devices and computer program product may be implemented with the following methods, systems, devices and computer program products disclosed in the following U.S. Patent Applications, U.S. Patents, and PCT International Patent Applications that are hereby incorporated by reference herein and co-owned with the assignee:

PCT International Application Serial No. PCT/US2005/013792, filed April 21, 2005, entitled "Method, System, and Computer Program Product for Evaluation of the Accuracy of Blood Glucose Monitoring Sensors/Devices,"

U.S. Patent Application No. 11/578,831, filed October 18, 2006 entitled "Method, System and Computer Program Product for Evaluating the Accuracy of Blood Glucose Monitoring Sensors/Devices;"

PCT International Application Serial No. PCT/US01/09884, filed March 29 2001, entitled "Method, System, and Computer Program Product for Evaluation of Glycemic Control in Diabetes Self-Monitoring Data;"

U.S. Patent No. 7,025,425 B2 issued April 11, 2006, entitled "Method, System, and Computer Program Product for the Evaluation of Glycemic Control in Diabetes from Self-Monitoring Data;"

U.S. Patent Application No. 11/305,946 filed December 19, 2005 entitled "Method, System, and Computer Program Product for the Evaluation of Glycemic Control in Diabetes from Self-Monitoring Data" (Publication No. 20060094947);

PCT International Application Serial No. PCT/US2003/025053, filed August 8, 2003, entitled "Method, System, and Computer Program Product for the Processing of Self-Monitoring Blood Glucose (SMBG) Data to Enhance Diabetic Self-Management;"

U.S. Patent Application No. 10/524,094 filed February 9, 2005 entitled "Managing and Processing Self-Monitoring Blood Glucose"(Publication No. 2005214892);

PCT International Application Serial No PCT/US2006/033724, filed August 29, 2006, entitled "Method for Improvising Accuracy of Continuous Glucose Sensors and a Continuous Glucose Sensor Using the Same;"

PCT International Application No. PCT/US2007/000370, filed January 5, 2007, entitled "Method, System and Computer Program Product for Evaluation of Blood Glucose Variability in Diabetes from Self-Monitoring Data;"

U.S. Patent Application No. 11/925,689, filed October 26, 2007, entitled "For Method, System and Computer Program Product for Real-Time Detection of Sensitivity Decline in Analyte Sensors;"

PCT International Application No. PCT/US00/22886, filed August 21, 2000, entitled "Method and Apparatus for Predicting the Risk of Hypoglycemia;"

U.S. Patent No. 6,923,763 B1, issued August 2, 2005, entitled "Method and Apparatus for Predicting the Risk of Hypoglycemia;" and

PCT International Patent Application No. PCT/US2007/082744, filed October 26, 2007, entitled "For Method, System and Computer Program Product for Real-Time Detection of Sensitivity Decline in Analyte Sensors."

Blood glucose self-monitoring devices are the current standard observational practice in diabetes, providing routine SMBG data that serve as the main feedback enabling patients to maintain their glycemic control. The aspects of the present invention system, method and computer program code of the present invention can, but not limited thereto, enhance existing SMBG devices by introducing data interpretation components capable of evaluating temporal glucose patterns of hyperglycemia and hypoglycemia, increased glucose variability, and inefficient self-monitoring.

Contemporary SMBG devices currently provide only general information to the patient, limited to BG readings and certain simple statistics, such as average. There are no existing pattern recognition methods that could be used for comparison.

In summary, while the present invention has been described with respect to specific embodiments, many modifications, variations, alterations, substitutions, and equivalents will be apparent to those skilled in the art. The present invention is not to be limited in scope by the specific embodiment described herein. Indeed, various modifications of the present invention, in addition to those described herein, will be apparent to those of skill in the art from the foregoing description and accompanying drawings. Accordingly, the invention is to be considered as limited only by the spirit and scope of the following claims, including all modifications and equivalents.

Still other embodiments will become readily apparent to those skilled in this art from reading the above-recited detailed description and drawings of certain exemplary embodiments. It should be understood that numerous variations, modifications, and additional embodiments are possible, and accordingly, all such variations, modifications, and embodiments are to be regarded as being within the spirit and scope of this application. For example, regardless of the content of any portion (e.g., title, field, background, summary, abstract, drawing figure, etc.) of this application, unless clearly specified to the contrary, there is no requirement for the inclusion in any claim herein or of any application claiming priority hereto of any particular described or illustrated activity or element, any particular sequence of such activities, or any particular interrelationship of such elements. Moreover, any activity can be repeated, any activity can be performed by multiple entities, and/or any element can be duplicated. Further, any activity or element can be excluded, the sequence of activities can vary, and/or the interrelationship of elements can vary. Unless clearly specified to the contrary, there is no requirement for any particular described or illustrated activity or element, any particular sequence or such activities, any particular size, speed, material, dimension or frequency, or any particularly interrelationship of such elements. Accordingly, the descriptions and drawings are to be regarded as illustrative in nature, and not as restrictive. Moreover, when any number or range is described herein, unless clearly stated otherwise, that number or range is approximate. When any range is described herein, unless clearly stated otherwise, that range includes all values therein and all sub ranges therein. Any information in any material (e.g., a United States/foreign patent, United States/foreign patent application, book, article, etc.) that has been incorporated by reference herein, is only incorporated by reference to the extent that no conflict exists between such information and the other statements and drawings set forth herein. In the event of such conflict, including a conflict that would render invalid any claim herein or seeking priority hereto, then any such conflicting information in such incorporated by reference material is specifically not incorporated by reference herein.

**Claims**

1. A method for identifying and/or predicting patterns of hyperglycemia, hypoglycemia, high glucose variability or ineffective testing of a user, said method comprising:

   (a) acquiring a plurality of SMBG data points;
   (b) classifying said SMBG data points within periods of time with predetermined durations;
   (c) evaluating glucose values or glucose variability in each period of time; and
   (d) indicating risk of hyperglycemia, hypoglycemia or high variability.

2. The method of claim 1, wherein said evaluation comprises:

   determining individual deviations towards hyperglycemia or hypoglycemia, based on said glucose values;
   determining a composite probability in each said period of time based on individual and absolute deviations; and
   comparing said composite probability in each period of time against a preset threshold.

3. The method of claim 2, wherein the determination of said deviations comprises calculating the average and standard deviation of SMBG for each said period of time.

4. The method of claim 2, wherein the determination of said deviations comprises calculating deviation contrasts for each said period of time.

5. The method of claim 4, wherein said deviation contrasts are computed as

$$t_k = \frac{X_k - \bar{X}}{\sqrt{\dfrac{SD^2}{N} + \dfrac{SD_k^2}{N_k}}} \, .$$

where $X_k$ represents the average SMBG readings in period of time k, $\bar{X}$ represents the mean of all the SMBG readings, SD represents the standard deviation of all SMBG readings, $SD_k$ represents the standard deviation of SMBG readings in period of time k, $N$ represents the total number of SMBG readings, and $N_k$ represents the number of SMBG readings in period of time $k$.

**6.** The method of claim 4, wherein said deviation contrasts are computed as

$$t_k = \sqrt{N}\,\frac{X_k - Y_k}{SD1}$$

where $Y_k$ is the average of the mean SMBG readings in the periods of time other than $k$, $X_k$ represents the average SMBG readings in period of time k, and $SD1$ represents an estimate of the standard deviation of $X_k$-$Y_k$.

**7.** The method of claim 2, wherein said composite probability comprises the probability of exceeding or being lower than an absolute threshold and the probability of exceeding or being lower than a relative personal threshold.

**8.** The method of claim 2, wherein said composite probability is computed as

$$CP_k(\alpha l) = P_k(\alpha l).\Phi(t_k)$$

where $P_k(\alpha 1)$ represents the probability of average SMBG in each said period of time to exceed or be lower than a preset threshold level $\alpha 1$, $\Phi(t_k)$ represents the probability of said average SMBG data in each said period of time to be higher or lower than average SMBG data of rest of said periods of time.

**9.** The method of claim 8, wherein said $\Phi(t_k)$ is the distribution function of a central normal distribution.

**10.** The method of claim 2, wherein said composite probability is computed as

$$CP_k(\alpha l) = P_k(\alpha l).\Phi(t_k)$$

where $P_k(\alpha 1)$ represents the probability of average SMBG in each said period of time to exceed or be lower than preset threshold level $\alpha 1$, $\Phi(t_k)$ represents the probability of said average SMBG data in each said period of time to be higher or lower than a grand mean.

**11.** The method of claim 10, wherein said $\Phi(t_k)$ is the distribution function of a central normal distribution.

**12.** The method of claim 2, wherein the calculation of said composite probabilities comprises calculating the probability of said average SMBG data in each said period of time to be higher or lower than average SMBG data of rest of said periods of time.

**13.** The method of claim 2, wherein the calculation of said composite probabilities comprise calculating the probability of said average SMBG data in each said period of time to be higher or lower than a grand mean.

**14.** The method of claim 1, wherein said evaluation comprising:

determining individual probability of each period of time having higher variability than other said periods of time;
determining an overall marker of variability with or without transforming said SMBG data points according to a transforming function; and
comparing said individual probability of each said period of time and said overall marker of variability against preset thresholds.

**15.** The method of claim 14, wherein said transforming function is computed as

$$f(BG, a, b) = c. \, [(ln \, (BG \,))^a - b\}]$$

where if BG is measured in mg/dl, then $a=1.084, b=5.381, c=1.509$ and if BG is measured in mmol/l, then $a=1.026, b=1.861$ and $c=1.794$.

**16.** The method of claim 14, wherein said determination of individual probability comprises of calculating at least one risk deviation for at least some of the each of the transformed plurality of SMBG data points.

**17.** The method of claim 16, wherein said risk deviations comprise of calculating ratios of standard risk deviations.

**18.** The method of claim 17, wherein said standard risk deviations are computed as

$$RSD_k^2 = \frac{1}{N_k - 1} \sum_{i=1}^{N_k} (f(X_{ki}) - f\overline{X}_k)^2 \text{, where } f\overline{X}_k = \frac{1}{N_k} \sum_{i=1}^{N_k} fX_{ki} \, .$$

where $RSD_k$ represents the risk standard deviation of SMBG readings in period of time k, $\overline{X}_k$ represents the average SMBG readings in period of time $k$, $X_{ki}$ represents the number of readings that fell into period of time k on day $i$ of the last 30 days of SMBG readings, $N$ represents the total number of SMBG readings, and $N_k$ represents the number of SMBG readings in period of time k.

**19.** The method of claim 17, wherein said ratio has approximately central normal distribution and is computed as

$$Z_k = 5*(RSD_k / RSD - 1)$$

where $RSD_k$ represents the risk standard deviation of SMBG readings in period of time k, and $RSD$ represents the risk standard deviation of all SMBG readings.

**20.** The method of claim 14, wherein said probability of a period of time having higher variability than other periods of time is computed as

$$P \, (Z_k > 0) = \Phi(Z_k),$$

wherein $\Phi$ is the distribution function of central normal distribution.

**21.** The method of claim 14, wherein said overall marker of variability comprises of an ADRR.

**22.** The method of claim 21, wherein said ADRR is computed as

$$ADRR = \frac{1}{M} \sum_{i=1}^{M} [LR^i + HR^i] \, ,$$

where $LR^i$ represents a maximal hypoglycemic risk value for period of time with a predetermined duration $i$, $HR^i$ represents a maximal hyperglycemic risk value for period of time with predetermined duration $i$, $LR^i + HR^i$ represents a calculated risk range for a period of time with predetermined duration $i$, and the plurality of blood glucose data points are collected on periods of time with predetermined duration $i = 1, 2, ..., M$.

23. The method of claim 1, wherein said plurality of SMBG readings comprises SMBG data from about two to six weeks of monitoring together with the time of each reading.

24. The method of claim 1, wherein each said period of time has a predetermined number of SMBG data points.

25. The method of claim 24, wherein said predetermined number of SMBG data points is at least about five for each said period of time.

26. The method of claim 1, wherein said periods of time comprises splitting twenty-four hour days into time bins with predetermined durations.

27. The method of claim 26, wherein said predetermined durations is between two to eight hours.

28. The method of claim 26, wherein said predetermined durations is fewer than twenty-four hours.

29. The method of claim 1, wherein said subsequent period of time comprises a next period of time.

30. The method of claim 1, wherein indication of said risk for hyperglycemia, hypoglycemia or high blood glucose variability comprises issuing a message indicating a pattern of high or low blood sugar or high blood glucose variability for a subsequent period of time.

31. The method of claim 30, wherein said message indicating a pattern of high or low blood glucose or higher blood glucose variability is received immediately by a user prior to said subsequent period of time.

32. The method of claim 30, wherein said subsequent period of time comprises a next period of time.

33. The method of claim 1, wherein indication of said risk for hyperglycemia, hypoglycemia or higher variability comprises issuing a message indicating a pattern of high or low blood sugar or high variability within about 24 hours of said acquisition of plurality of SMBG data points.

34. The method of claim 1, wherein indication of said risk for hyperglycemia, hypoglycemia or high variability comprises issuing a message indicating a pattern of high or low blood sugar or high variability within about 12 hours of said acquisition of plurality of SMBG data points.

35. The method of claim 1, wherein indication of said risk for hyperglycemia, hypoglycemia or high variability comprises issuing a message indicating a pattern of high or low blood sugar or high variability within about 6 hours of said acquisition of plurality of SMBG data points.

36. The method of claim 1, wherein indication of said risk for hyperglycemia, hypoglycemia or high variability comprises issuing a message indicating a pattern of high or low blood sugar or high variability at the completion of the above claimed steps.

37. The method of claim 1, wherein the indication of said risk of hyperglycemia or hypoglycemia occurs near contemporaneously to the latest SMBG testing.

38. A method for identifying patterns of ineffective testing of a user, said method comprising:

   (a) acquiring plurality of SMBG data points;
   (b) classifying said SMBG data points within periods of time with predetermined durations;
   (c) calculating the percentage of SMBG readings in each said period of time;
   (d) comparing percentage against preset thresholds; and
   (e) indicating ineffective testing for said period of time.

39. The method of claim 38, wherein the indication of ineffective testing comprisies issuing a message indicating ineffective testing for said period of time.

40. The method of claim 38, wherein said message comprises warning about insufficient testing if said percentage is below a preset threshold.

**41.** The method of claim 38, wherein said message comprises warning about excessive testing if said percentage is above a preset threshold.

**42.** A system for identifying and/or predicting patterns of hyperglycemia or hypoglycemia of a user, said system comprising:

an acquisition module for acquiring a plurality of SMBG data points; and
a processor programmed to:

carry out the method of any one of claims 1-13 and 23-37 with the exception of step (a).

**43.** A system for identifying and/or predicting patterns of high glucose variability of a user, said system comprising:

an acquisition module for acquiring a plurality of SMBG data points; and
a processor programmed to:

carry out the method of any one of claims 1 and 14-36 with the exception of step (a).

**44.** A system for identifying and/or predicting patterns of ineffective testing of a user, said system comprising:

an acquisition module for acquiring a plurality of SMBG data points; and
a processor programmed to:

carry out the method of any one of claim 38-41 with the exception of step (a).

**45.** A computer program product comprising a computer useable medium having computer program logic for enabling at least one processor in a computer system to carry out the system of claims 42, 43 or 44.

**46.** The method of claim 1, system of claim 43 and computer program product of claim 45, wherein indication of said risk for high variability comprises issuing a message indicating a pattern of high variability occurs near contemporaneously to said acquisition of plurality of said SMBG data points.

**47.** The method of claim 1, system of claim 42 or 43 and computer program product of claim 45, wherein the indication of said risk of hyperglycemia, hypoglycemia or high variability comprises issuing a message indicating a pattern of hyperglycemia, hypoglycemia or high variability wherein the issuance occurs contemporaneously to a user-initiated action or at a predetermined time.

**48.** The method of claim 1, system of claim 42 or 43 and computer program product of claim 43, wherein indication of said risk for hyperglycemia, hypoglycemia or high variability comprises issuing a message indicating a pattern of hyperglycemia, wherein the issuance occurs in real time or at a predetermined time.

**49.** The method, system and computer program product of claim 47, wherein said user-initiated action is the acquisition of one or more SMBG data points.

**50.** The method, system and computer program product of claim 47 or 48, wherein said predetermined time is within 24 hours of the acquisition of one or more SMBG data points.

## FIG. 1

Step 1: Internal
Condition, BG Level

Low       High

Probability of   Symptoms /
Difficulty   Perception ,
Given Internal Condition

Step 2: Symptom
Perception/Awareness.

YES          NO

Probability of
Appraisal ,
Given Perception of
Internal Condition

Step 3: Appraisal,
Estimated BG

YES          NO

Probability of a
Decision ,
Given Appraisal of
Internal Condition

Step 4: Judgement
Raise (or Lower) BG

Yes          No

## FIG. 2

**FIG. 3**

| | 11 PM-7 AM | 7-11 AM | 11 AM-3 PM | 3-7 PM | 7-11 PM |
|---|---|---|---|---|---|
| # readings | 20 | 17 | 30 | 18 | 28 |
| Mean BG (& SD) | 320.0 | 183.6 | 155.3 | 217.9 | 231.8 |
| Deviation t-Statistic | 2.66 | -1.00 | -2.94 | 0.01 | 0.48 |
| Deviation Probability | >0.99 | 0.16 | <0.01 | 0.50 | 0.69 |
| P(BG > 200) | 0.65 | 0.35 | 0.33 | 0.39 | 0.54 |
| Composite Probability | 0.65 | 0.06 | <0.01 | 0.20 | 0.37 |

**FIG. 4**

| | 11 PM-7 AM | 7-11 AM | 11 AM-3 PM | 3-7 PM | 7-11 PM |
|---|---|---|---|---|---|
| # readings | 24 | 14 | 14 | 18 | 10 |
| Mean BG (& SD) | 230.5 | 153.0 | 205.6 | 298.0 | 272.1 |
| Deviation t-Statistic | -0.08 | -2.89 | -0.88 | 2.61 | 1.27 |
| Deviation Probability | 0.47 | <0.01 | 0.19 | >0.99 | 0.90 |
| P(BG > 200) | 0.58 | 0.29 | 0.36 | 0.83 | 0.90 |
| Composite Probability | 0.27 | <0.01 | 0.07 | 0.83 | 0.81 |

# FIG. 5

| | 11 PM-7 AM | 7-11 AM | 11 AM-3 PM | 3-7 PM | 7-11 PM |
|---|---|---|---|---|---|
| # readings | 16 | 21 | 19 | 9 | 21 |
| Mean BG | 173.5 | 165.8 | 197.0 | 214.3 | 247.0 |
| Deviation t-Statistic | -0.78 | -1.28 | -0.09 | 0.52 | 1.77 |
| Deviation Probability | 0.78 | 0.90 | 0.54 | 0.30 | 0.04 |
| P(BG ≤ 70) | 0.25 | 0.33 | 0.00 | 0.00 | 0.10 |
| Composite Probability | 0.20 | 0.30 | 0.00 | 0.00 | 0.00 |

# FIG. 6

| | 11 PM-7 AM | 7-11 AM | 11 AM-3 PM | 3-7 PM | 7-11 PM |
|---|---|---|---|---|---|
| # readings | 22 | 41 | 37 | 22 | 38 |
| SD (risk)* | 0.74 | 0.74 | 0.83 | 1.02 | 0.88 |
| Deviation z-Statistic | -0.64 | -0.62 | -0.11 | 1.04 | 0.16 |
| Deviation Probability | 0.26 | 0.27 | 0.46 | 0.85 | 0.56 |

\* The same test can be based on SD of BG, which will make the variability profile more sensitive to hyperglycemic excursions and less sensitive to hypoglycemia.

# FIG. 7

| Subject A: | 11 PM-7 AM | 7-11 AM | 11 AM-3 PM | 3-7 PM | 7-11 PM |
|---|---|---|---|---|---|
| # readings | 6 | 46 | 57 | 41 | 37 |
| % readings | 3.2% | 24.6% | 30.5% | 21.9% | 19.8% |

| Subject B: | 11 PM-7 AM | 7-11 AM | 11 AM-3 PM | 3-7 PM | 7-11 PM |
|---|---|---|---|---|---|
| # readings | 11 | 30 | 24 | 5 | 56 |
| % readings | 8.7% | 23.8% | 19.0% | 4.0% | 44.4% |

# FIG. 8

FIG. 9

EP 1 956 371 A2

**FIG. 10**

FIG. 11

**FIG. 12**

EP 1 956 371 A2

## REFERENCES CITED IN THE DESCRIPTION

### Patent documents cited in the description

- US 87640206 P
- US 2007000370 W
- US 2003025053 W
- US 5267152 A, Yang
- US 5086229 A, Rosenthal
- US 4975581 A, Robinson
- US 5139023 A, Stanley
- US 5036861 A, Sembrowich
- US 5076273 A, Schoendorfer
- US 5140985 A, Schroeder
- US 5279543 A, Glikfeld
- WO 9600110 A, Tamada
- US 6144869 A, Berner

- US 5851186 A, Wood
- US 2005013792 W
- US 57883106 A
- US 0988401 W
- US 7025425 B2
- US 30594605 A
- US 52409405 A
- US 2006033724 W
- US 92568907 A
- US 0022886 W
- US 6923763 B1
- US 2007082744 W

### Non-patent literature cited in the description

- **AABY SVENDSEN P ; LAURITZEN T ; SOEGARD U ; NERUP J.** Glycosylated Haemoglobin and Steady-State Mean Blood Glucose Concentration in Type 1 (Insulin-Dependent) Diabetes. *Diabetologia,* 1982, vol. 23, 403-405
- **BOLLI G.** How to ameliorate the problem of hypoglycemia in intensive as well as nonintensive treatment of Type 1 diabetes. *Diabetes Care,* 1999, vol. 22 (2), B43-B52
- **CLARKE WL ; COX D ; GONDER-FREDERICK LA ; CARTER W ; POHL SL.** Evaluating the clinical accuracy of self-blood glucose monitoring systems. *Diabetes Care,* 1987, vol. 10, 622-628
- **COX DJ ; GONDER-FREDERICK LA ; POLONSKY W ; SCHLUNDT D ; JULIAN D ; CLARKE WL.** A multicenter evaluation of blood glucose awareness training-II. *Diabetes Care,* 1995, vol. 18, 523-528
- **COX DJ ; GONDER-FREDERICK LA ; POLONSKY W ; SCHLUNDT D ; JULIAN D ; KOVATCHEV BP ; CLARKE WL.** Blood Glucose Awareness Training (BGAT-II): Long term benefits. *Diabetes Care,* 2001, vol. 24, 637-642
- **COX DJ ; KOVATCHEV B ; JULIAN D ; GONDER-FREDERICK LA ; POLONSKY WH ; SCHLUNDT DG ; CLARKE WL.** Frequency of severe hypoglycemia in IDDM can be predicted from self-monitoring blood glucose data. *Journal of Clinical Endocrinology and Metabolism,* 1994, vol. 79, 1659-1662

- **COX DJ ; KOVATCHEV BP ; CLARKE WL ; GONDER-FREDERICK LA ; TRAJANOSKI Z.** Accuracy of Home Blood Glucose Meters for Prediction of Severe Hypoglycemia (SH). *Diabetes,* 1999, vol. 48 (1), A101
- **CRANSTON I ; LOMAS J ; MARAN A ; MACDONALD I ; AMIEL S.** Restoration of hypoglycemia unawareness in patients with long duration insulin-dependent diabetes mellitus. *Lancet,* 1994, vol. 344, 283-287
- **CRYER PE.** Hypoglycaemia: The limiting factor in the glycaemic management of type I and type II diabetes. *Diabetologia,* 2002, vol. 45, 937-948
- **CRYER PE.** Iatrogenic hypoglycemia as a cause of hypoglycemia-associated autonomic failure in IDDM: A vicious cycle. *Diabetes,* 1992, vol. 41, 255-260
- **CRYER PE.** Hypoglycemia: The Limiting factor in the management of IDDM. *Diabetes,* 1994, vol. 43, 1378-1389
- **DAGOGO-JACK S ; RATTARASARN C ; CRYER PE.** Reversal of hypoglycemia unawareness, but not defective glucose counterregulation, in IDDM. *Diabetes,* 1994, vol. 43, 1426-1434
- **FANELLI C ; EPIFANO L ; RAMBOTTI A et al.** Meticulous prevention of hypoglycemia (near-) normalizes magnitude and glycemic thresholds of neuroendocrine responses to, symptoms of, and cognitive function during hypoglycemia in intensively treated patients with IDDM of short duration. *Diabetes,* 1993, vol. 42, 1683-1689

- **FANELLI CG ; PAMPANELLI S ; EPIFANO L et al.** Longterm recovery from unawareness, deficient counterregulation and lack of cognitive dysfunction during hypoglycemia following institution of rational intensive therapy in IDDM. *Diabetologia,* 1994, vol. 37, 1265-1276
- **GOLD AE ; FRIER BM ; MACLEOD KM ; DEARY IJ.** A structural equation model for predictors of severe hypoglycaemia in patients with insulin-dependent diabetes mellitus. *Diabet Med,* 1997, vol. 14, 309-315
- **GOLD AE ; DEARY IJ ; FRIER BM.** Recurrent severe hypoglycaemia and cognitive function in type I diabetes. *Diabet Med,* 1993, vol. 10, 503-508
- **HENDERSON JN ; ALLEN KV ; DEARY IJ ; FRIER BM.** Hypoglycemia in insulin-treated Type 2 diabetes: frequency, symptoms and impaired awareness. *Diabet Med,* 2003, vol. 20, 1016-1021
- **INOUYE K ; SHUM K ; CHAN O ; MATHOO JMR ; MATTHEWS SG ; VRANIC M.** Effects of recurrent hyperinsulinemia with and without hypoglycemia on counterregulation in diabetic rats. *Am JPhysiol Endocrinol Metab,* 2002, vol. 282, E1369-E1379
- **KINSLEY BT ; WEINGER K ; BAJAJ M ; LEVY CJ ; SIMONSON DC ; QUIGLEY M ; COX DJ ; JACOBSON AM.** Blood glucose awareness training and epinephrine responses to hypoglycemia during intensive treatment in type 1 diabetes. *Diabetes Care,* 1999, vol. 22, 1022-1028
- **KOVATCHEV BP ; COX DJ ; FARHY LS ; STRAUME M ; GONDER-FREDERICK LA ; CLARKE, WL.** Episodes of Severe Hypoglycemia in Type 1 Diabetes are Preceded, and Followed, within 48 Hours by Measurable Disturbances in Blood Glucose. *J of Clinical Endocrinology and Metabolism,* 2000, vol. 85, 4287-4292
- **KOVATCHEV BP ; COX DJ ; GONDER-FREDERICK LA ; WL CLARKE.** Methods for quantifying self-monitoring blood glucose profiles exemplified by an examination of blood glucose patterns in patients with Type 1 and Type 2 Diabetes. *Diabetes Technol Ther,* 2002, vol. 4 (3), 295-303
- **KOVATCHEV BP ; COX DJ ; GONDER-FREDERICK LA ; WL CLARKE.** Symmetrization of the blood glucose measurement scale and its applications. *Diabetes Care,* 1997, vol. 20, 1655-1658
- **KOVATCHEV BP ; COX DJ ; GONDER-FREDERICK LA ; YOUNG-HYMAN D ; SCHLUNDT D ; CLARKE WL.** Assessment of risk for severe hypoglycemia among adults with IDDM: Validation of the Low Blood Glucose Index. *Diabetes Care,* 1998, vol. 21, 1870-1875
- **KOVATCHEV BP ; COX DJ ; GONDER-FREDERICK LA ; CLARKE WL.** Low Blood Glucose Index Predicts Occurrence of Severe Hypoglycemia Among Adults with IDDM. *Diabetes,* 1998, vol. 47 (1), A107
- **KOVATCHEV BP ; COX DJ ; GONDER-FREDERICK LA ; CLARKE WL.** Transforming the Blood Glucose Scale: Statistical and Clinical implications. *Diabetes,* 1997, vol. 46 (1), A268
- **KOVATCHEV BP ; COX DJ ; GONDER-FREDERICK LA ; SCHLUNDT D ; WL CLARKE.** Stochastic model of self-regulation decision making exemplified by decisions concerning hypoglycemia. *Health Psychology,* 1998, vol. 17, 277-284
- **KOVATCHEV BP ; COX DJ ; KUMAR A ; GONDER-FREDERICK LA ; WL CLARKE.** Algorithmic Evaluation of Metabolic Control and Risk of Severe Hypoglycemia in Type 1 and Type 2 Diabetes Using Self-Monitoring Blood Glucose (SMBG) Data. *Diabetes Technology and Therapeutics,* 2003, vol. 5 (5), 817-828
- **KOVATCHEV BP ; STRAUME M ; COX DJ ; FARHY LS.** Risk analysis of blood glucose data: A quantitative approach to optimizing the control of insulin dependent diabetes. *J of Theoretical Medicine,* 2001, vol. 3, 1-10
- **KOVATCHEV BP ; COX DJ ; STRAUME M ; FARHY LS.** Estimating the Speed of Blood Glucose Transitions and its Relationship with Severe Hypoglycemia. *Diabetes,* 1999, vol. 48 (1), A363
- **KOVATCHEV BP ; OTTO E ; COX DJ ; GONDER-FREDERICK LA ; CLARKE WL.** Evaluation of a New Measure of Blood Glucose Variability in Diabetes. *Diabetes Care,* 2006, vol. 29, 2433-2438
- **LINCOLN NB ; FALEIRO RM ; KELLY C ; KIRK BA ; JEFFCOATE WJ.** Effect of long-term glycemic control on cognitive function. *Diabetes Care,* 1996, vol. 19, 656-658
- **MATHOO JMR ; SHI ZQ ; KLIP A ; VRANIC M.** Opposite effects of acute hypoglycemia and acute hyperglycemia on glucose transport and glucose transporters in perfused rat skeletal muscle. *Diabetes,* 1999, vol. 48, 1281-1288
- **MCAULAY V ; DEARY IJ ; FRIER BM.** Symptoms of hypoglycaemia in people with diabetes. *Diabet Med,* 2001, vol. 18, 690-705
- **REICHARD P ; PHIL M.** Mortality and treatment side effects during long-term intensified conventional insulin treatment in the Stockholm Diabetes Intervention study. *Diabetes,* 1994, vol. 43, 313-317
- **RYAN EA ; SHANDRO T ; GREEN K ; PATY BW ; SENIOR PA ; BIGAM D ; SHAPIRO AMJ ; VANTYGHEM MC.** *Diabetes,* 2004, vol. 53, 955-962
- **SANTIAGO JV.** Lessons from the Diabetes Control and Complications Trial. *Diabetes,* 1993, vol. 42, 1549-1554
- **SCHLICHTKRULL J ; MUNCK O ; JERSILD M.** *Acta Med Scand,* 1965, vol. 177, 95-102
- **SERVICE FJ ; MOLNER GD ; ROSEVEAR JW ; ACKERMAN E ; GATEWOOD LC ; TAYLOR WF.** *Diabetes,* 1970, vol. 19, 644-655

- The Diabetes Control and Complications Trial Research Group. Hypoglycemia in the Diabetes Control and Complications Trial. *Diabetes,* 1997, vol. 46, 271-286
- The Diabetes Control and Complications Trial Research Group. The effect of intensive treatment of diabetes on the development and progression of long-term complications of insulin-dependent diabetes mellitus. *N Engl J Med,* 1993, vol. 329, 978-986
- The diabetes research in children network (DirecNet) study group. A multicenter study of the accuracy of the One Touch® Ultra® home glucose meter in children with Type 1 diabetes. *Diabetes Technol Ther,* 2003, vol. 5, 933-942
- UK Prospective Diabetes Study Group (UKPDS). Intensive blood-glucose control with sulphonylureas or insulin compared with conventional treatment and risk of complications in patients with type 2 diabetes. *Lancet,* 1998, vol. 352, 837-853

- **KOVATCHEV BP ; COX DJ ; GONDER-FREDERICK LA ; WL CLARKE.** Symmetization of the Blood Glucose Measurement Scale and Its Applications. *Diabetes Care,* 1997, vol. 20, 1655-1658
- **KOVATCHEV BP ; STRAUME M ; COX DJ ; FARHI LS.** Risk Analysis of Blood Glucose Data: A Quantitative Approach to Optimizing the Control of Insulin Dependent Diabetes. *J of Theoretical Medicine,* 2001, vol. 3, 1-10
- **KOVATCHEV BP ; COX DJ ; GONDER-FREDERICK LA ; WL CLARKE.** Methods for quantifying self-monitoring blood glucose profiles exemplified by an examination of blood glucose patterns in patients with Type 1 and Type 2 Diabetes. *Diabetes Technology and Therapeutics,* 2002, vol. 4 (3), 295-303
- **KOVATCHEV BP ; COX DJ ; KUMAR A ; GONDER-FREDERICK LA ; CLARKE WL.** Algorithmic Evaluation of Metabolic Control and Risk of Severe Hypoglycemia in Type 1 and Type 2 Diabetes Using Self-Monitoring Blood Glucose (SMBG) Data. *Diabetes Technology and Therapeutics,* 2003, vol. 5 (5), 817-828